# EUROPEAN PATENT SPECIFICATION

(11) **EP 1 864 607 B1**
(45) Date of publication and mention of the grant of the patent: **27.07.2011**
(21) Application number: 07011272.7
(22) Date of filing: 08.06.2007
(51) Int. Cl.: A61B 1/12, A61B 1/04, A61L 2/24

(54) **System for managing cleaning and disinfecting steps for endoscope**
System zur Verwaltung der Reinigungs- und Desinfektionsmaßnahmen eines Endoskops
Système de gestion des étapes de nettoyage et de désinfection d'un endoscope

(30) Priority: 07.06.2006 JP 2006158811
(43) Date of publication of application: 12.12.2007
(73) Proprietor: OLYMPUS MEDICAL SYSTEMS CORP., Tokyo 151-0072 (JP)
(72) Inventor: Noguchi, Toshiaki, Tokyo 151-0072 (JP); Suzuki, Eiri, Tokyo 151-0072 (JP); Kobayashi, Kenichi, Tokyo 151-0072 (JP); Hasegawa, Hitoshi, Tokyo 151-0072 (JP); Suzuki, Shintaro, Tokyo 151-0072 (JP); Ogawa, Akihisa, Tokyo 151-0072 (JP)
(74) Representative: von Hellfeld, Axel

(56) References cited:
- EP-A- 0 945 140
- EP-A- 1 153 568
- EP-A- 1 155 654
- US-A- 4 862 872
- US-A1- 2002 161 460
- US-A1- 2004 052 679
- US-B1- 6 436 032

## Description

### Background of the Invention

### (The Field of the Invention)

The present invention relates to an endoscopic cleaning and disinfecting management system and a management method for managing cleaning and disinfecting steps for used endoscopes.

### (Related Art)

In the medical field, endoscopes have now become one of indispensable medical instruments. Endoscopes are used for mainly examining and treating the insides of cavities of an object being examined. In general, an endoscope is equipped with a long tubular insertion member, called insertion tube, which is flexible to be inserted along body cavities. Inside the insertion tube, various endoscopic ducts (i.e., channels) are formed along a longitudinal axial direction thereof. These endoscopic ducts include a suction duct which serves as a forceps channel. Hence, once this insertion tube is inserted into a body cavity, not only the outer surface of the insertion tube but also the inside thereof, that is, its endoscopic ducts (channels) become dirty because of filthy materials such as bodily fluid adhering thereon. Hence it is necessary that the outer surface of the insertion tube as well as the endoscopic ducts should be cleaned and disinfected sufficiently after use.

To clean endoscopes, Japanese Patent Laid-open Publication No. 11-76145 exemplifies an endoscope washer. This washer is provided with a brush inserting duct to allow a washing brush to be inserted into endoscopic ducts, so that that the ducts can be cleaned with the washing brush.

However this conventional endoscope is still confronted with a problem which could not be overcome sufficiently, even if the brush inserting tube is employed. The problem is that, as the time goes by longer after use of an endoscope, it becomes more difficult to remove filthy materials such as bodily fluid adhereing on, especially, the endoscopic ducts. Although there is such a difficulty, the conventional technique is totally silent about how to cope with such a difficulty as to the cleaning, which difficulty is resultant from a longer elapse of time after use of the endoscope. Hence in the conventional, cleaning results often fluctuate regarding how degree the endoscope has been cleaned. In some cases, the filthy materials cannot be removed fully. Accordingly, an operator who is responsible for reprocessing work (including brushing, cleaning, disinfecting, rinsing and drying steps) should pay much attention to cleaning results, whereby management of the reprocessing work (especially cleaning and disinfecting steps) burdens heavily on the operator.

Document EP 0 945 140 A2 concerns an apparatus for washing and disinfecting/sterilizing an endoscope. The apparatus comprises a washing vessel capable of housing the endoscope. In one embodiment, the endoscope comprises an IC memory in which data of the endoscope including the kind of endoscope, the date of manufacture, the date of delivery, the number of inspections, date of inspection and inspection time can be stored.

Document US 4,862,872 concerns an endoscope and an endoscope washing apparatus. The endoscope comprises a memorizing part for memorizing an operation time of the endoscope.

Document US 2002/0161460 concerns an automatic washer disinfector apparatus comprising a holder having an RFID transponder attached to a side surface of the holder and an RFID transmission/reception circuit main body provided on a side surface of a wagon bearing the holder.

Document US 2004/0052679 A1 concerns a reusable instrument, such as an endoscope. Recognition data can be read from the reusable instrument in order to identify the identity of a client using the reusable instrument and for charging the client based on data associated with the client.

Document EP 1153 568 A2 concerns a system for managing cleaning information for an endoscope. The system comprises a cleaning apparatus for sterilizing/cleaning an endoscope used in an endoscope apparatus and a printer for printing a sticker to be stuck on the endoscope after being cleaned indicating that the endoscope has been cleaned.

Document EP 1 155 654 A1 concerns an endoscope system having an endoscope including a columnar transponder having readable characteristic information for discriminating a plurality of endoscopes, an endoscope cleaner including a nameplate type transponder having readable characteristic information for discriminating endoscope cleaners, and a nameplate type transponder having readable characteristic information for discriminating a user operating the endoscope or the endoscope cleaner.

### Summary of the Invention

The present invention has been made in view of the foregoing difficulty, and has an object to provide a managing system which is able to manage the cleaning and disinfection steps for endoscopes such that the endoscopes can be deaned well in a stable manner, regardless of how long the time elapses after use of the endoscopes.

In order to achieve the above object, the managing system according to the present invention is a managing system for managing not only an endoscope washer-disinfector performing at least cleaning and disinfection with an insertion tube of an endoscope, the insertion tube being inserted into at least a duct of an object being examined, but also an endoscope apparatus performing an endoscopic examination using the endoscope. The system comprises the features of claim 1.

### Brief Description of the drawings

In the accompanying drawings:
Fig. 1 is a perspective appearance view showing an endoscope system according to a first embodiment of the present invention;
Fig. 2 is a view showing the configuration of an endoscope apparatus;
Fig. 3 is a view showing the configuration of an endoscope;
Fig. 4 is a view showing the structure of a distal end face of a universal cable;
Fig. 5 is a view outlining the structure of a remote control;
Fig. 6 is a block diagram showing the configuration of a video processor;
Fig. 7 is a plan view showing a washing bath of an endoscope washer-disinfector;
Fig. 8 is a block diagram showing the electrical configuration of the endoscope washer-disinfector;
Fig. 9 is a flowchart showing the flow of processing for an endoscopic examination performed by a video processor;
Fig. 10 exemplifies a screen which requests cleaning and disinfection steps to be executed again;
Fig. 11 is a flowchart showing the flow of processing for cleaning, disinfecting, rinsing and drying steps executed by the endoscope washer-disinfector;
Fig. 12 is a view exemplifying a screen used for setting how the cleaning and disinfecting steps are performed depending on the length of a period of time elapsing from the finish of an endoscopic examination;
Fig. 13 is a view exemplifying a screen which requests pre-cleaning;
Fig. 14 is a view exemplifying another screen used for setting how the cleaning and disinfecting steps are performed depending on the length of a period of time elapsing from the finish of an endoscopic examination;
Fig. 15 is a view exemplifying a screen which notifies that it is not recognized that the pre-cleaning has been peformed;
Fig. 16 is a view showing the appearance of the insertion tube of an endoscope;
Fig. 17 is a view showing the appearance of the insertion tube of another endoscope;
Fig. 18 is a perspective view showing a tray in which the insertion tube of an endoscope is accommodated, which endoscope is according to a second embodiment of the present invention;
Fig. 19 is a flowchart showing the flow of processing executed by a video processor in the second embodiment;
Fig. 20 exemplifies a screen requiring that the cleaning and disinfection should be done again;
Fig. 21 is a flowchart showing a flow of processing for cleaning, disinfecting, rinsing and drying steps executed by an endoscope washer-disinfector;
Fig. 22 exemplifies a screen requiring that pre-cleaning should be done;
Fig. 23 is a perspective appearance view showing an endoscope system according to a third embodiment of the present invention;
Fig. 24 is a view showing the rear side of the endoscope system;
Fig. 25 is a view showing the internal structure of an endoscope;
Fig. 26 is a block diagram showing an electrical configuration of a video processor;
Fig. 27 is a block diagram showing an electrical configuration of an endoscope washer-disinfector;
Fig. 28 is a block diagram showing electrical connections of an endoscope system;
Fig. 29 is a flowchart showing the flow of processing executed by the video processor; and
Fig. 30 is a flowchart showing the flow of processing executed by the endoscope washer-disinfector.

### Detailed Description of the Preferred Embodiments

Referring to the accompanying drawings, various embodiments of the present invention will now be described.

### (First embodiment)

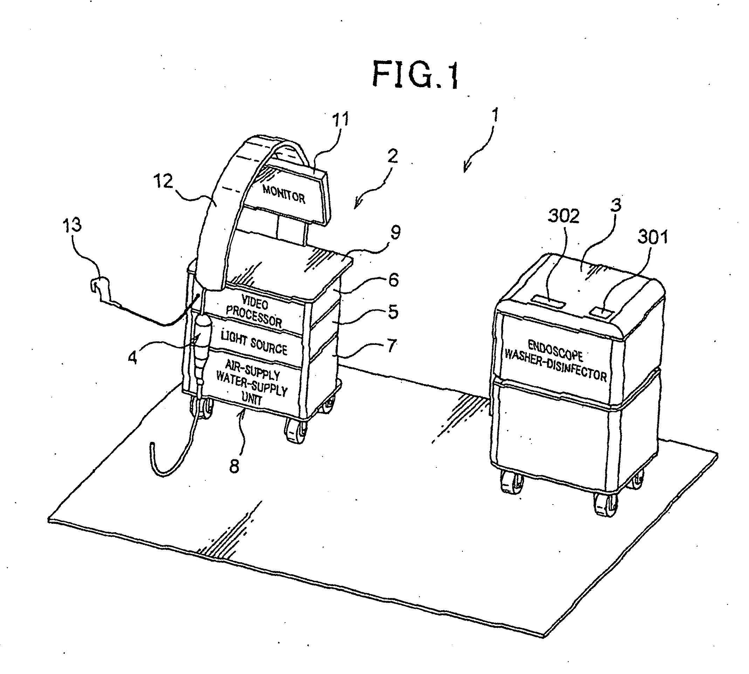

Referring to Figs. 1-17, an endoscope system according to a first embodiment will now be described.

Fig. 1 shows an outer appearance of an endoscope system 1. This endoscope system 1 is functionally provided with a managing system according to the present invention, in which the managing system is for managing the cleaning and disinfection work of an endoscope. That is, the endoscope system 1 is capable of functionally performing management for the cleaning and distinction work. As shown in Fig. 1, the endoscope system 1 functionally includes an endoscope apparatus 2 and an endoscope washer-disinfector 3.

Of those components, the endoscope apparatus 2 is provided with an endoscope 4 (hereinafter also called "scope" in some situations) and an arm 12 having flexibility for sustaining the endoscope 4. The endoscope apparatus 2 is further provided with a light source 5 to supply illuminating light to the endoscope 4, in addition to a video processor 6, an air-supply water-supply unit 7, and a monitor 11. In terms of outlined operations, the video processor 6 is adapted to apply signal processing to signals imaged by imaging elements (not shown) mounted in the endoscope 4 and to produce endoscopic images depending on the processed image signals. The air-supply water-supply unit 7 is adapted to supply air and water to the endoscope 4 and perform a sucking-in operation with the endoscope 4. The monitor 11, which serves as display means, is composed of, for example, a liquid crystal monitor to display endoscopic images produced by the video processor 6.

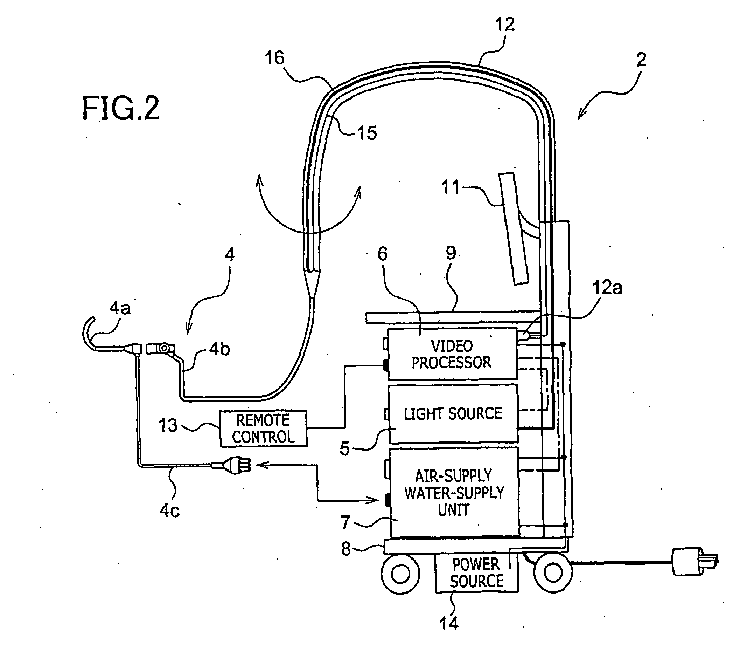

As shown in Fig. 2, the video processor 6, light source 5, and air-supply water-supply unit 7 are mounted on a cart to be stacked on one another. The cart 8 is provided with a power source 14 beneath thereof, in which the power source 14 powers the video processor 6, light guide 5, and air-supply water-supply unit 7.

The cart 8 has a pole on an upper part of which the monitor 11 is mounted in a tiltable manner in the back-and-forth and lateral directions. At a predetermined position lower than the monitor 11, but sill highly located, a work plate 9 is fixedly secured to the pole. On the work plate 9, there are a keyboard and a mouse serving as a pointing device, though not shown. Using these input devices, an operator (including a doctor) is able to give various data to the video processor 6, light source 5, and air-supply water-supply unit 7.

The video processor 6 is formed to be detachably connected with a remote control 13. Using operator's operations at this remote control makes it possible that, during an examination of a body cavity of a patient using the endoscope 4, bending operations of the endoscope 4 are controlled as will be described later and images from the endoscope 4 are controlled as to their releasing and freezing actions.

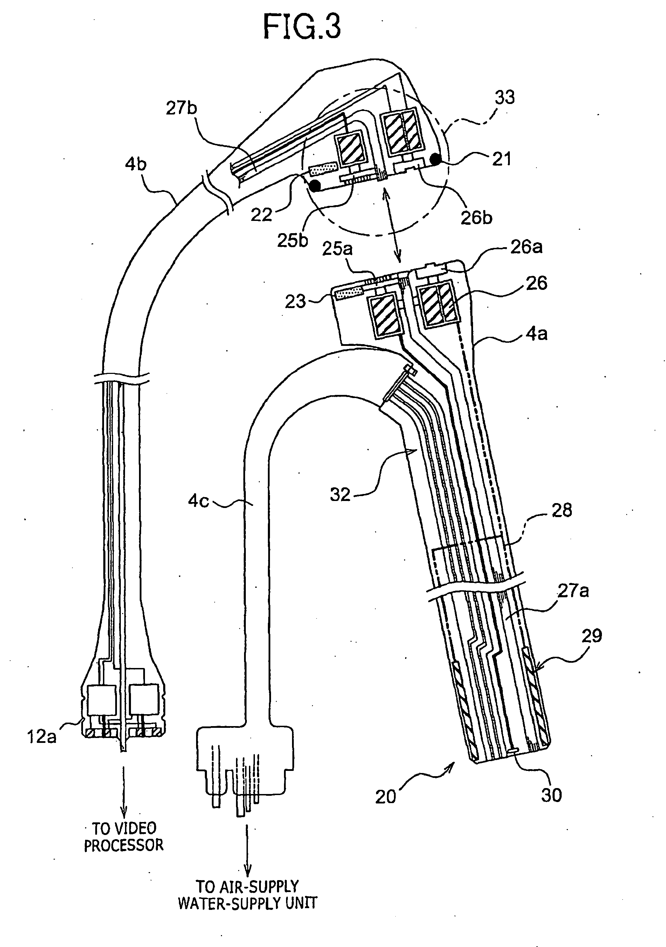

In this endoscope apparatus 2, the endoscope 2 and the video processor 6 are mutually electrically connected by a signal cable 15, and there is a light guide cable 16 to transmit to the endoscope 4 the illuminating light from the light source 5. These cables 15 and 16 are placed in the arm 12 to be passed therethrough. As shown in Fig. 3, inside a base end part of the arm 12, there is provided an arm connector 12a which terminates both base end parts of the signal cable 15 and light guide cable 16. Thus the arm connector 12a is linked with the video processor 6.

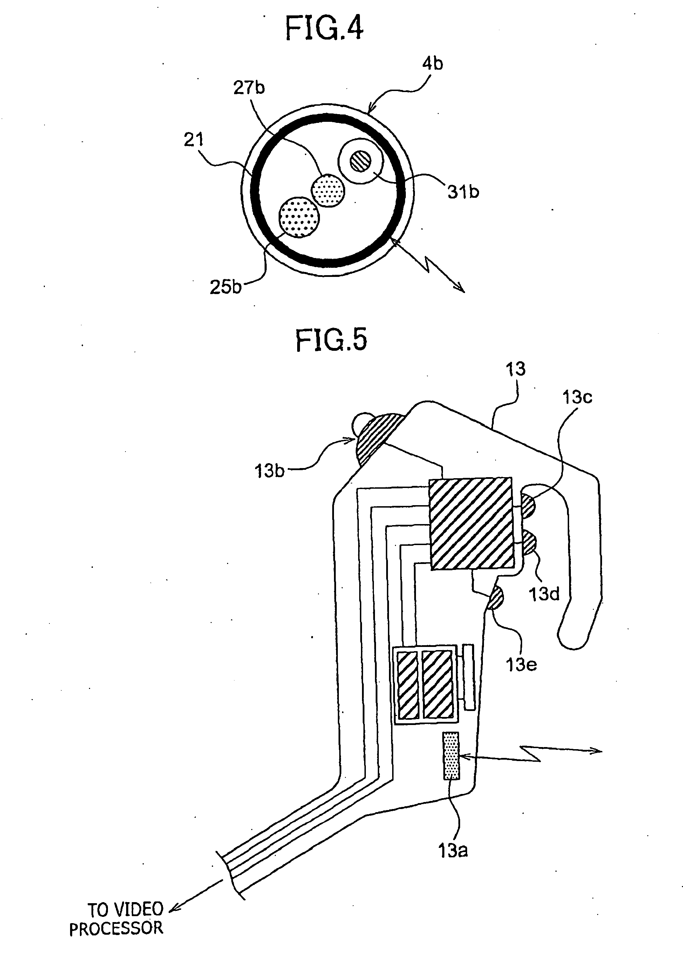

As shown in Fig. 4, the endoscope 4 is provided with a long tubular member 4a (hereinafter simply referred to as "insertion tube") to be inserted into a body cavity of an object to be examined for the purpose of endoscopic examinations and treatments, a universal cable 4b detachably connected to this insertion tube 4a, and a duct tube 4c. The universal cable 4b is arranged along inside or outside the arm 12. The arm connector 12a, which is connected with the universal cable 4b, is electrically connected with the video processor 6. This allows the universal cable 4b to electrically be connected to the video processor 6 via a signal cable 15. Further, within the arm 12, a light guide cable 16 is arranged to transmit to the universal cable 4b illuminating light coming from the light source 5. The arm connector 12a is connected to the video processor 6 in a detachable manner.

As shown in Fig. 3, the insertion tube 4a is equipped with imaging elements 30 positioned at a distal end thereof (i.e., an end oppositely positioned to a base end which is nearer to the operator), wherein the imaging elements are composed of for example a CCD sensor or a C-MOS sensor). The insertion tube 4a has a base end portion which enables both distal ends of the universal cable 4b and the duct tube 4c to detachably be connected to the insertion tube 4a.

The insertion tube 4a is provided with a group of ducts composed of plural ducts for various purposes, including ducts for air-supply water-supply, of which distal ends are opened outside in the surface of the distal end of the insertion tube 4a. These plural ducts are directed along a longitudinal axis which can imaginarily be set within the insertion tube 4a. The base end faces of the group of ducts are linked with the distal end face of the duct tube 4c. The base end part of the duct tube 4c is detachably coupled with the air-supply water-supply unit 7. By way of example, the duct tube 4c is a disposal type of tube which is disposed of whenever an endoscopic examination has been finished.

As above, with the distal end part of the universal cable 4b connected to the base end part of the insertion tube 4a, the video processor 6 is able to drive and control the operations of the imaging elements 30 disposed at the distal end part of the insertion tube 4a, via the signal cable 15.

In the distal end part of the insertion tube 4a, there is arranged a bendable portion 29 with a bendable piece. The bendable portion 29 (i.e., the distal end part) of the insertion tube 4a is bendable in the up-and-down and right-and-left directions by pulling and releasing a plurality of wires 28 (e.g., four wires) coupled with the bendable portion 29. The wires 28 are operated by a driving unit 26 disposed at the base end part of the insertion tube 4a.

With the universal cable 4b connected with the insertion tube 4a, the universal cable 4b is also connected with the video processor 6, which allows the video processor 6 to drive and control the driving unit 26 via the signal cable 15.

That is, when the universal cable 4b is connected with the insertion tube 4a, connectors 25a and 25b disposed at the base end part of the insertion tube 4a are physically and electrically connected with connectors 25b and 26b disposed at the distal end part of the universal cable 4b.

Thus, in this connected state, electric signals from the video processor 6 are transmitted to the imaging elements 30 via the signal cable 15 and connectors 25a and 25b. In this connected states, the electric signals from the video processor 6 are also transmitted to the driving unit 26 via the signal cable 15 and connectors 26a and 26b.

Meanwhile the insertion tube 4a is equipped with a light guide 27a disposed therein to be along the insertion axis thereof. The light guide 27a has a distal end face which is water-tightly opened to the outside via a not-shown objective lens in the distal end face of the insertion tube 4a. The light guide 27a has a base end face optically connected to a distal end face of a light guide 27b inserted in along the universal cable 4b without deteriorating the optical characteristics. This connection is realized when the universal cable 4b is connected with the base end of the insertion tube 4. Furthermore, when the base end of the universal cable 4b is connected to the arm connector 12a of the arm 12, the base end face of the light guide 27b is optically connected to the distal end face of the light guide 16 disposed in along the arm 12, without deterioration in the optical characteristics.

Hence, with the insertion tube 4 connected with the universal cable 4b, the illuminating light from the light source 5 is transmitted to the distal end of the insertion tube 4a via the light guide 16 in the arm 12, the light guide 27b in the universal cable 4b, and the light guide 27a in the insertion tube 4a. The illuminating light reached the distal end of the insertion tube 4a is projected to an object positioned in front of the distal end thereof via the not-shown objective lens system incorporated within the distal end of the insertion tube 4a).

In the present embodiment, in both the base end part of the insertion tube 4a and the distal end part of the universal cable 4b, RFID (Radio Frequency IDentification) devices 23 and 22, which serve as time memorizing means, are disposed, respectively. These RFID devices 23 and 22 are non-contact authentication systems that make use of IC chips.

Thus, each of the RFID devices 22 and 23 has a memory (not shown) writable and readable for endoscopic examination system data (hereinafter simply referred to as "examination data.") The "examination data" includes i) scope ID indicating the ID of the endoscope 4 (i.e., a type name, identification information such as manufacture's serial number, information indicating duct characteristics, and others) and ii) various device-inherent data (i.e., the number of times of use, information indicating repair history, data of CCD characteristics, and others); system data (i.e., the date of a examination, a start time of the examination, an finish time of the examination, data indicating devices used in the examination, a person who made the examination, and others); and history data of reprocessing work (i.e., the date of reprocessing work, an finish time instant of the reprocessing work, the number of times of reprocessing work, information indicating an endoscope washer-disinfector used, a person who made the reprocessing work, and others). The reprocessing work includes an automatic brushing, cleaning, disinfecting, rinsing and drying steps in a programmed order.

In the RFID device 23 incorporated in the insertion tube 4a, the scope ID indicative of the insertion tube 4a itself and the examination data corresponding to the scope ID are written.

In addition, the universal cable 4b has an RFID antenna 21 embedded within the distal end thereof. The RFID antenna 21 is produced such that the antenna 21 is able to transmit and receive data to and from the foregoing RFID devices 22 and 23 without wires and to transmit and receive data to and from the video processor 6 via the signal cable 15, i.e., with wires. Incidentally, in Fig. 3, the RFID antenna 21 is depicted as black circles, but in effect, as shown in Fig. 4 showing a part circled by a dashed line in Fig. 3, the antenna 21 has an annular shape.

As shown in Fig. 5, the remote control 13 has an outer surface on which a plurality of switches are provided. The switches includes a joystick type of bending switch 13b commanding drive of the driving unit 26 via the video processor 6 and a plurality of switches 13c to 13e respectively commanding various control actions such as releasing and freezing actions of the video processor 6.

Additionally, the remote control 13 contains an RFID device 13a therein. In the similar way to the above RFID sections 22 and 23, this RFID section 13a has a memory which is able to read and write examination data for each scope ID. Making the remote control 13 approaching the universal cable 4b allows the RFID device 13a to transmit and receive data to and from the RFID antenna 21 without wires.

In this way, the reason why the remote control 13 is additionally provided with RFID device 13 is that the remote control 13 needs to be cleaned and disinfected in some cases.

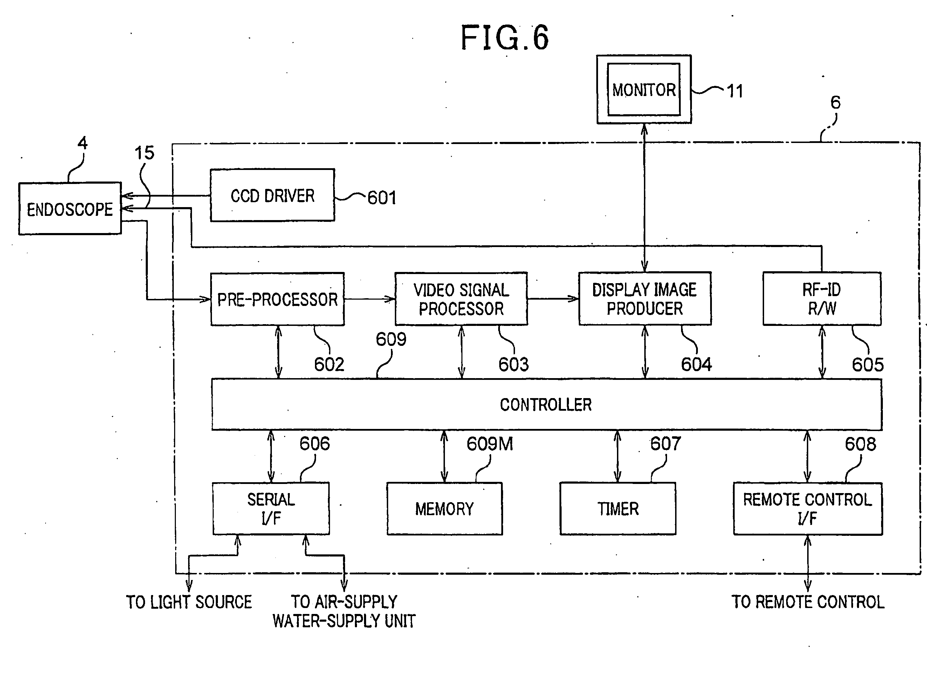

As shown in Fig. 6, the video processor includes a CCD driver 601 driving the imaging elements 30 incorporated in the insertion tube 4a of the endoscope 4 and a pre-processor 602. This pre-processor 602 is formed to perform pre-processing on image signals from the imaging elements 30, which pre-processing includes correlative double sampling, luminance adjustment, and A/D conversion.

The video processor 6 further includes a video signal processor 603 and a display image producer 604. The video signal processor 603 is formed to apply signal processing to digital image signals preprocessed by the pre-processor 602, in which the signal processing is for example white balance processing, color adjustment, and gamma correction. The display image producer 604 receives the image signal undergoing the signal processing carried out by the video signal processor 603, and displays images on the monitor 11 using the received image signals.

The video processor 6 further includes an RFID R/W 605 driving the RFID antenna 21 disposed in the universal cable 4b of the endoscope 4, so that the RFID antenna 21 is able to transmit and receive examination data to and from the RFID devices 22 and 23 and 13a. Additionally, the video processor 6 includes a remote control I/F (interface) 608 receiving a command signal from the remote control 13 and a serial I/F 606 handling serial communication with the light source 5 and the air-supply water-supply unit 7 (for example, communication on RS-232C).

In addition to the above circuit members, the video processor 6 includes a controller 609 with a CPU (central processing unit), in which the controller 609 controls the foregoing circuit members based on the software processing executed by the CPU. This controller 609 is a main part of control actions executed in the video processor 6. In general, the controller 609 manages overall control actions in the circuit members as well as controls various control actions in terms of time using a timer 607 to log history data of controlled states in a data storage 609M.

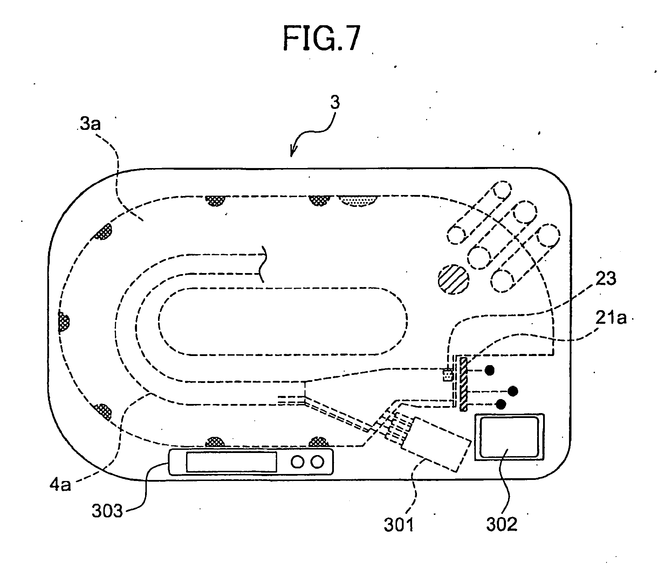

As shown in Fig. 7, the endoscope washer-disinfector 3 has an upper face on and in which there are equipped with an operation panel 303 and an LCD display unit 302 (refer to Fig. 1). The LCD display unit 302 has a touch panel function, which gives a user an interface to the endoscope washer-disinfector 3.

In addition, in the endoscope washer-disinfector 3, there is equipped with a washing bath 3a with a predetermined depth. For example, the insertion tube 4a is mounted in the washing bath 3 to be positioned in place therein. An RFID antenna 21a is disposed in the endoscope washer-disinfector 3 at a position outside, but near the washing bath 3a, wherein the position faces a connection face of the insertion tube 4a and the connection surface is connected to the universal cable 4b. The RFID antenna 21a is disposed such that the connection face of the insertion tube 4a, which is connected to the duct tube 4c, is directed approximately perpendicular to a brush advancing and pulling-back direction of a washing brush unit 301 arranged outside and near the washing bath 3a.

Using the examination data stored in the RFID device 23 of the insertion tube 4a, the endoscope washer-disinfector 3 drives in a controlled manner the washing brush unit 301 so that the unit 301 brushes the insides of a group of ducts 32 (refer to Fig. 3) of the insertion tube 4a for a predetermined period of time. Then the endoscope washer-disinfector 3 supplies various types of liquid, such as washing liquid and disinfecting liquid, into the washing bath 3a for washing and disinfecting steps for a predetermined period of time, respectively. Then the endoscope washer-disinfector 3 performs rinsing and drying steps as well.

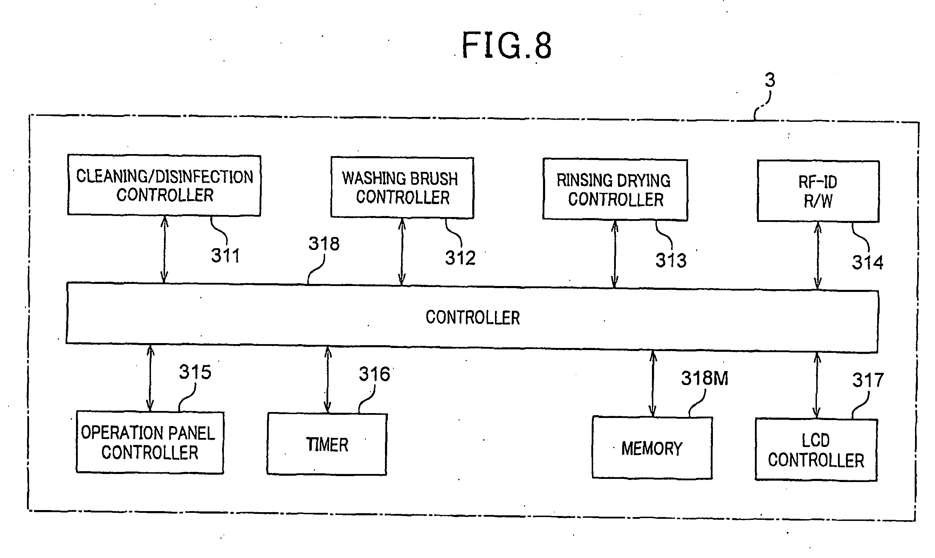

As shown in Fig. 8, the endoscope washer-disinfector 3 comprises, in addition to a washing/disinfecting controller 311 controlling cleaning and disinfecting steps, a washing brush controller 312, a rinsing/drying controller 313, an operation panel controller 315, and an LCD controller 317. The washing brush controller 312 controls brushing work carried out by the washing brush unit 301, while the rising/drying controller 313 controls rinsing and drying steps. The operation panel controller 315 controls the operations of the operation panel 303. The LCD controller 317 controls touch panel functions and display actions of the LCD display unit 302.

The endoscope washer-disinfector 3 is equipped with an RFID R/W 314 serving as RFID reading and reading means. This RFID R/W 314 drives the RFID antenna 21a to transmit and receive examination data to and from the RFID device 23 incorporated in the insertion tube 4a.

The endoscope washer-disinfector 3 is equipped with a controller 318 controlling the above circuit members. Especially the controller 318, which serves as means for measuring a current time instant, uses a time 316 to manage time for various types of control and log history data of controlled states in a data storage 318M. In the present embodiment, the controller 318 functionally realizes washing/disinfecting step control means and time comparing means.

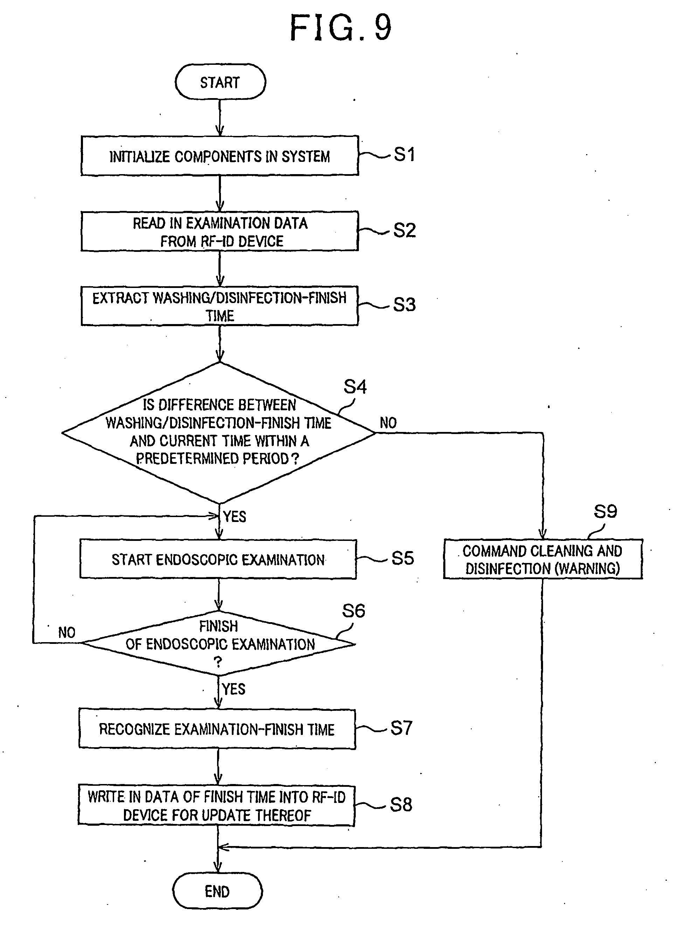

Referring to Figs. 9 - 15, the operations performed by the above endoscope system 1 according to the present embodiment will now be described.

Prior to an endoscopic examination, an operator connects the insertion tube 4a with both the universal cable 4b and the duct tube 4c to assemble the endoscope 4. And the operator connects the universal cable 4b to the video processor 6 and the duct tube 4c to the air-supply water-supply unit 7. The remote control 13 is connected to the video processor 6 by the operator.

In this connected state, the operator turns on the power of the video processor 6, light source 5, and air-supply water-supply unit 7, so that the endoscope apparatus 2 is ready for endoscopic examinations.

In this state, the video processor 6 starts its processing, which is shown in Fig. 9. As shown, the controller 609 (refer to Fig. 6) initializes the respective components thereof at step S1.

Then at step S2, the controller 609 reads examination data out of RFID device 23 via the RFID antenna 21 of the universal cable 4b (refer to Fig. 3).

The controller 609 proceeds to step S3, where the controller extracts, from the read-out examination data, "cleaning/disinfection-finish time data," which includes a date on which a cleaning and disinfection work was done and a finish time instant of the cleaning and disinfection work, which data is related to the history data of the cleaning and disinfecting steps.

As will be described later, the "cleaning/disinfection-finish time data" is updated by the endoscope washer-disinfector 3 whenever the insertion tube 4a is cleaned and disinfected. When insertion tubes are shipped from manufacturers, a predetermined default value is recorded as the "cleaning/disinfection-finish time data."

Then the controller 609 proceeds to step S3, where the controller 609 makes a comparison between "the extracted cleaning/disinfection-finish time data" and "the current date and time (simply "current time data")" indicated by the timer 607. That is, it is determined whether or not a difference between the extracted cleaning/disinfection-finish time instant and the current time instant is within a predetermined period of time (for example, 4 hours).

If the determination at step S3 reveals that the difference is within the predetermined period of time, the controller 609 recognizes that it is possible to perform an endoscopic examination using the endoscope 4 now being connected to the endoscope apparatus 2. Thus the controller 609 performs the processing for the endoscopic examination at step S5, and continues the examination until it is determined that the examination has been finished, with repeating steps S5 and S6.

When it is recognized that the examination has been finished, the controller 609 proceeds to step S7, where the controller 609 memorizes "the current time data", which is indicated by the timer 607, as information showing the "cleaning/disinfection-finish time data."

The controller 609 proceeds to step S8, where the controller 609 overwrites the memorized "cleaning/disinfection-finish time data" as an examination finish time in the examination data in the RFID device 23 via the RFID antenna 21 of the universal cable 4b, every scope ID. This allows the examination time to be updated, before completing the processing.

In the process at step S8, the controller 609 also writes the memorized examination finish time into both the RFID device 22 of the universal cable 4 and the RFID device 13a of the remote control 13, respectively, as the examination finish time in the examination data stored therein.

The reason why information about the examination finish time is written into the RFID devices 22 and 13a other than the RFID device 23 is that, if necessary, the system is ready for enabling the universal cable and/or remote control to be cleaned and disinfected, additionally to or separately from the insertion tube. In the present embodiment, however, the insertion tube is a sole objective to be cleaned and disinfected.

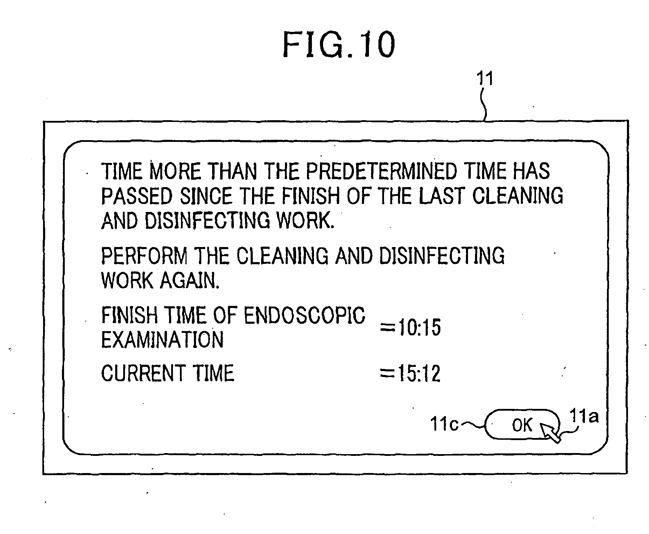

Meanwhile when the difference between the cleaning/disinfection-finish time instant and the current time instant exceeds the predetermined period of time, the controller 609 performs its processing at step S9 to display on the monitor 11 an error notice screen as shown in Fig. 10. By this error notice, an error message saying that "time more than a predetermined period of time has passed since the finish of the last cleaning and disinfecting work. It is required that the insertion tube should be cleaned and disinfected again." is notified of the user as a warning. Though not shown, the controller 609 is configured to respond to an operator's operation to click an OK button 11c with a pointer 11a in the error notice screen. The operator can issue such a command using the mouse, for example. In response to the operator's operation, the controller will close the processing.

In the present embodiment, the video processor 6 compares in real time the cleaning/disinfection-finish time data with the current time data for the insertion tube 4a to be targeted, prior to an endoscopic examination. Hence it is possible to use, without fail, only the endoscope 4 having the insertion tube 4a of which elapse time from the finish of the last cleaning and disinfecting work is still within the predetermined period of time.

How to preprocess (i.e., how to clean and infect) the endoscope 4, which has been used for the endoscopic examination, will now be described in terms of its time management.

The reprocessing work is performed by the endoscope washer-disinfector 3. In the present embodiment, the insertion tube 4a of the endoscope 4 is a target to be reprocessed (i.e., cleaned and disinfected). However, devices such as universal cable and/or remote control other than the insertion tube may be reprocessed as well.

An operator sets the insertion tube 4a, which has been used for the endoscopic examination, in the washing bath 3a, before the power of the endoscope washer-disinfector 3 is put on. Responsively to this operation, this washer-disinfector 3 starts the reprocessing work.

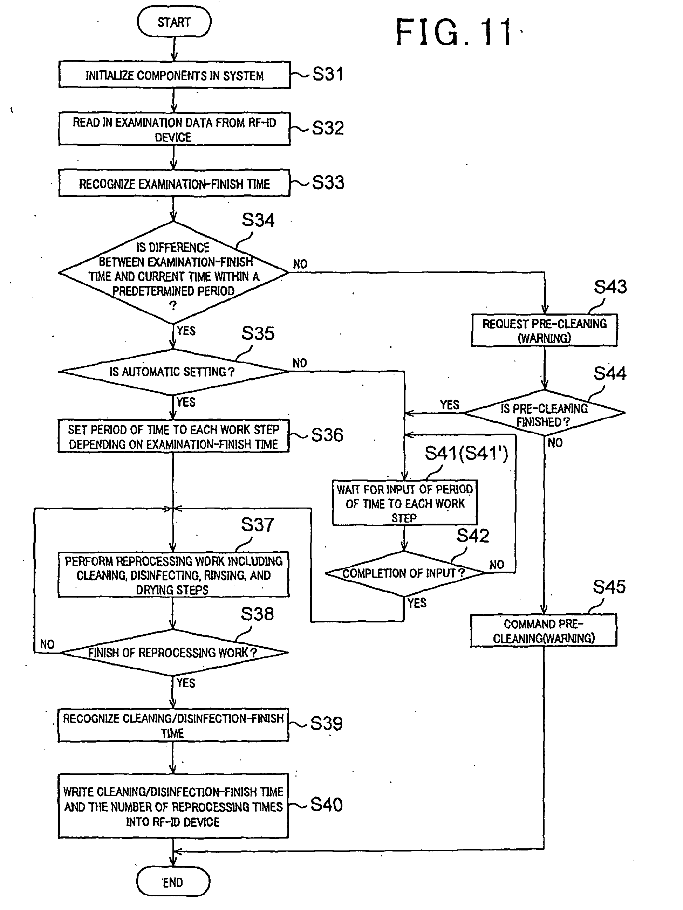

As shown in Fig. 11, when the reprocessing work is started, the controller 318 (refer to Fig. 8) of the endoscope washer-disinfector 3 initializes the respective components of the washer-disinfector 3 at step S31.

The controller 318 then proceeds to step S32, where the controller 318 reads in examination data from the RFID device 23 via the RFID antenna 21a of the endoscope washer-disinfector 3 (refer to Fig. 7).

The controller 318 then proceeds to step S33, where the controller 318 recognizes, from the read-in examination data, at least the date on which an endoscopic examination is performed and a time instant at which the endoscopic examination is finished (, simply "examination-finish time data" which is part of the system data.

This examination-finish time data is updated by the video processor 6 at a time when the endoscopic examination is finished, as described already. However, the insertion tube 4a is shipped at first from its manufacturer, a default code for the examination-finish time data is written in the insertion tube.

Then the controller 318 proceeds to step S34, where the controller 318 makes a comparison between the recognized examination-finish time data and the current time data counted by the timer 316. Practically this comparison is performed by determining whether or not a difference between the examination-finish time instant and the current time instant is within a predetermined period of time (for example, 30 minutes).

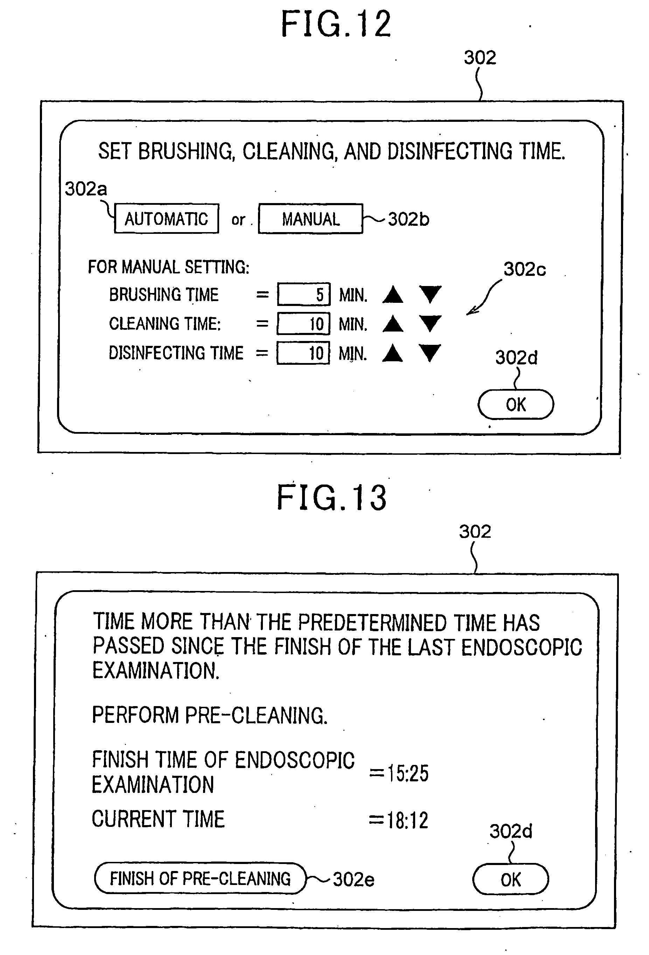

When it is determined at step S34 that the difference is within the predetermined period of time, the controller 318 proceeds to step S35 to display, on the LCD display unit 302 (refer to Fig. 7), a time setting screen exemplified in Fig. 12. And the controller 318 waits for operator's operations to be done through the touch panel function owned by the OCD display unit 302.

Concretely, when the operator clicks an "automatic" button 302a and an "OK" button 302d on the time setting screen shown in Fig. 12, the controller 318 moves its processing from step S35 to step S36. In contrast, when the operator clicks a "manual" button 302b and the "OK" button 302d, the controller 318 moves its processing from step S35 to step S41.

At step S36, on the basis of the examination-finish time data (i.e., the difference between the examination-finish time instant and the current time instant), the controller 318 calculates a period of time for brushing (brushing time) during which the cleaning brush unit 301 operates, a period of time for cleaning (cleaning time), and a period of time for disinfection (disinfection time), before proceeding to step S37.

Meanwhile, at step S41, a manual setting area 302c on the time setting screen shown in Fig. 12 becomes active and the controller 318 waits for operator's commands for setting the brushing time, cleaning time, and disinfection time from the manual setting area 302c. Until the operator clicks the OK button 302d, the controller 318 repeats the processes at steps S41 and S42. And when it is recognized at step S42 that the operator has clicked the OK button 302d, the processing is shifted to step S37. Incidentally, if the determination at step S34 is YES (i.e., within the predetermined period of time), the processing may be skipped automatically to step S37 from step S34.

At step S37, the controller 318 controls the reprocessing work including the brushing, cleaning, disinfecting, rinsing and drying steps. That is, the controller 318 controls the brushing, cleaning, and disinfecting steps based on the calculated or set brushing time, cleaning time, and disinfection time and also controls the rising and drying steps in a predetermined programmed order. At step S38, the controller 318 confirms the finish of those work steps, and continues repeating the processes at steps S37 and S38. On completion of confirming that all the work steps have been finished at step S38, the controller 318 escapes from the repeated processes, before proceeding to step S39.

At step S39, the controller 318 recognizes, i.e., memorizes current time data counted by the timer 316, as the cleaning/disinfection-finish time data.

After this memorization, the controller 318 proceeds to step S40, where the controller 318 overwrites, in the RFID device 23, the recognized cleaning/disinfection-finish time data as the cleaning/disinfection-finish time of the examination data via the RFID antenna 21a of the endoscope washer-disinfector 3 (refer to Fig. 7), every scope ID. Thus the cleaning/disinfection-finish time can be updated.

In addition, at step S40, data showing how many times the reprocessing work have been performed so far is incremented for update. Then the processing is terminated. By counting the number of times of the reprocessing work, a reference index showing that how deeply the insertion tube is deteriorated can be obtained.

By contrast, in cases where it is determined at step S34 that the difference between the examination-finish time instant and the current time instant reaches or exceeds the predetermined period of time, the processing will be done as follows.

In such a case, i.e., NO at step S34, the controller 318 executes the processing at step S43. At this step S43, the controller 318 makes the LCD display unit 302 represent thereon a screen requesting pre-cleaning serving as pre-reprocessing, as shown in Fig. 13. This representation gives an operator a warning that a considerably longer period of time (i.e., longer than the predetermined period of time) has already been passed since the date and time instant when the endoscopic examination was finished.

After this, at step S44, the controller determines whether or not the operator has clicked a "pre-cleaning finished" button 302e or the "OK" button 302e on the pre-cleaning request screen of the LCD display unit shown in Fig. 13, with the aid of the touch panel function thereof.

The pre-cleaning, which is pre-reprocessing, is manual reprocessing (manual work) requesting that operators manually brush, with the use of a washing brush, the outer surface of an insertion tube and the inside of the ducts of the insertion tube in a predetermined manner for a predetermined period of time.

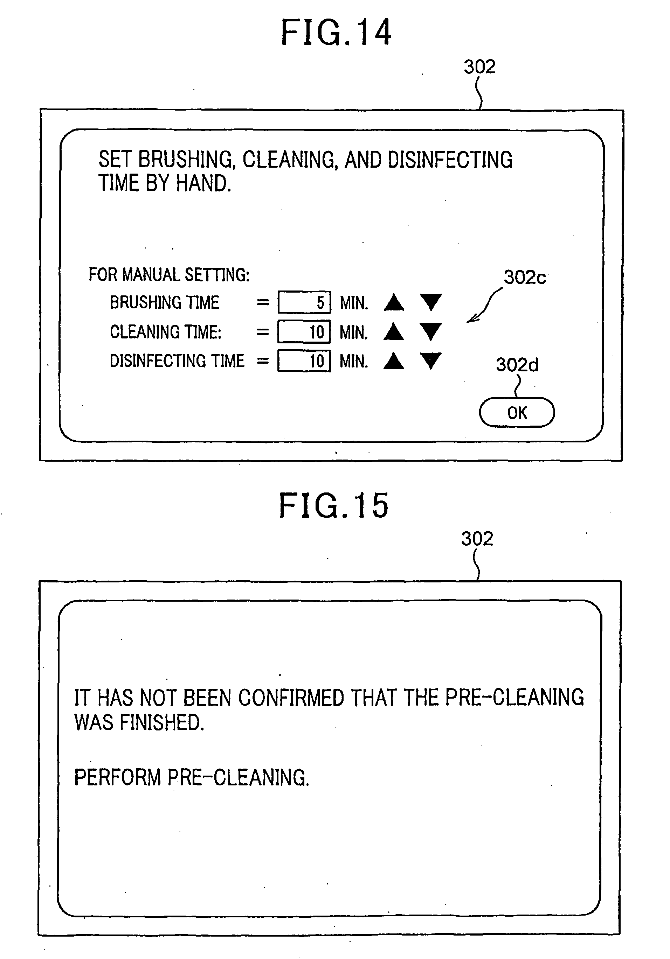

When it is determined at step S44 that the "pre-cleaning finished" button 302e has been clicked, the controller 318 allows the LCD display unit 302 to represent a screen (i.e., time setting screen) exemplified in Fig. 14, before going to step S41. Using the time setting screen, the operator can set, by hand, a desired time to each of the brushing, cleaning, and disinfecting times for the cleaning and disinfection.

Meanwhile, when it is determined at step S44 that the "OK" button 302e has been clicked, the LCD display unit 302 is allowed to represent a screen (warning screen) making a warning that the pre-cleaning should be done. This warning screen is exemplified in Fig. 15. Then the processing is terminated.

In this way, in the present embodiment, the endoscope washer-disinfector 3 is able to make a comparison between the examination-finish time instant of the insertion tube 4a being preprocessed and the current time instant. As a result of the comparison, if an elapse time from the finish of an endoscopic examination is still within a predetermined period of time (threshold), the brushing, cleaning and disinfecting steps are atomically performed in turns on the basis of automatically of manually set time periods.

Meanwhile, if the above elapse time has already reached or exceeded the predetermined period of time, a warning that the insertion tube 4a should be pre-cleaned is issued. Hence the operator should obey the warning and carry out the pre-cleaning.

Thus the insertion tube 4a can be brushed, cleaned, and disinfected in a reliable and effective fashion. In particular, depending on how long the time after use of the endoscope has elapsed, the insertion tube can be cleaned and disinfected. Specifically, in cases where the time equal to or more than a predetermined period of time has already passed since the last use, filthy materials on the insertion tube is more reluctant to be removed. In this case, the pre-cleaning is requested, so that the cleaning result is kept good always.

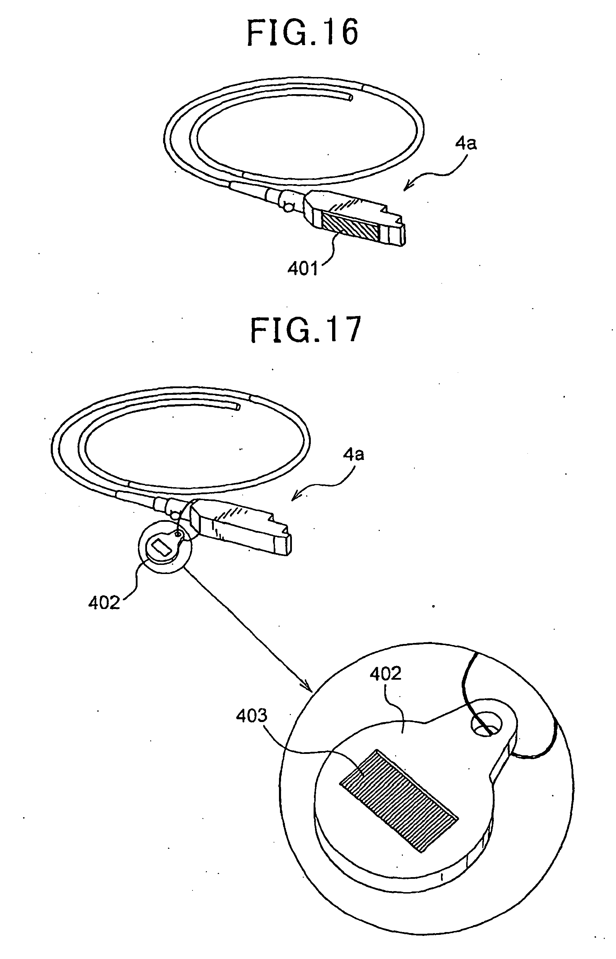

Modifications of the foregoing configurations can also be provided as follows. In the foregoing embodiment, the RFID device 23 is incorporated within the base end part of the insertion tube 4a. However, this is just an example, and as shown in Fig. 16, an RFID device 401 identical in functions to the RFID device 23 may be secured on the outer surface of the base end part of the insertion tube 4a. In this case, the RFID device 401 is allowed to have a display member such as known electronic paper which does not need power to sustain displayed contents. Such a display member can be used to display the examination-finish time and the cleaning/disinfection-finish time. By displaying the examination-finish time and the cleaning and disinfection time on the display member, such bits of temporal information can be visually confirmed with ease, prior to setting the insertion tube 4a.

Further, Fig. 17 is another example, where a tag 402 is detachably attached to the base end part of the insertion tube 4a. This tag 402 is provided with an RFID device identical in functions to the foregoing RFID device 23. In this structure, the RDID device 403 may have a known electronic paper as its display member, as mentioned above, which display member is formed to display the examination-finish time and the cleaning/disinfection-finish time.

Incidentally, in the managing system according to the foregoing first embodiment, the processes at steps S2 - S4 and S9 may be omitted from the processing. In other words, the processing is configured such that the examination-finish time is updated alone after the endoscopic examination. And the updated results may be reflected in managing the work in the cleaning and disinfection steps (as in Fig. 11) being performed based on an elapse time from the finish of the last examination. This makes it possible to surely manage the cleaning and disinfection work according to the temporal length elapsing from the finish of the last endoscopic examination.

### (Second embodiment)

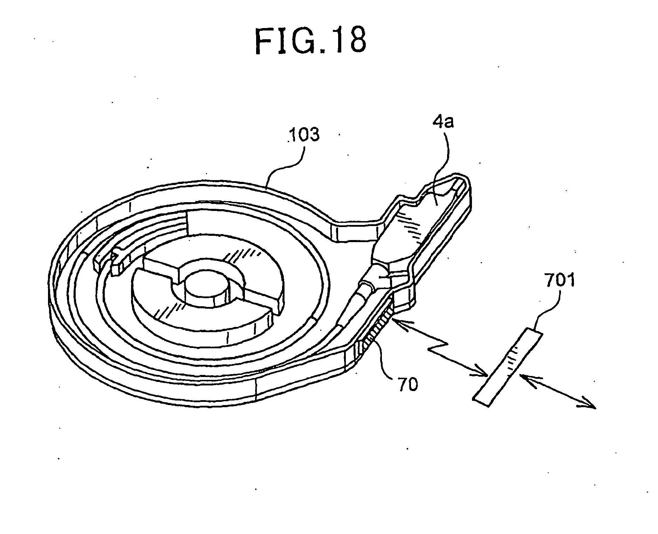

Referring to Figs. 18-20, a second embodiment of the present invention will now be described.

Since the configurations and functions of an endoscope system according to the second embodiment are mostly identical or similar to those of the first embodiment, only different parts from those of the first embodiment will now be described for the sake of a simplified explanation. The identical or similar components to those in the first embodiment will be given the same reference numerals.

In the present embodiment, as shown in Fig. 18, there is provided a tray 103 for endoscopes. The tray 103 is capable of accommodating the insertion tube 4a, so that the tray 103 is used such that, before and after an endoscopic examination, the insertion tube 4a is accommodated in the tray 103 for delivery, cleaning and disinfection, and storage.

This tray 103 can be loaded directly into the washing bath 3a of the endoscope washer-disinfector 3 (refer to Fig. 7). With the insertion tube 4a accommodated in the tray 103, the endoscope washer-disinfector 3 has the capability of brushing, cleaning and disinfecting the insertion tube 4a.

On the outer surface of the tray 103, an RFID device 70 is secured which is able to transmit and receive data to and from an RFID antenna 701 without wires. This RFID antenna 701, which is able to communicate with the device 70 and the video processor 6, is disposed on the arm 12 or the work plate 9 (refer to Fig. 1) or an appropriate place near those components 12 or 9.

In this configuration, the RFID device 70 is allowed to have a display member composed of a known electronic paper requiring no power to sustain displayed contents. In such a case, it is desirable that the display member is configured to display at least one of the examination-finish time and the cleaning/disinfection-finish time. If such temporal data are displayed on the display member such as electronic paper, an operator is able to visually confirm the temporal information before setting the insertion tube 4a.

The RFID antenna 701 may be an antenna disposed on the outside of the cart 8 or mounted at the distal end of the universal cable 4b. As long as the RFID antenna 701 is allowed to communicate with the RFID device 70 and located near the endoscope apparatus 2 or the cart 8, the RFID antenna 701 may be located arbitrarily. In the case of being located on the universal cable 4b, signals can be transmitted from the insertion tube 4a, to via the universal cable 4b, and to the video processor 6, and received through the opposite path thereto, without wires. However, depending on where to locate the antenna 701, signals may be transmitted between the video processor 6 and the insertion tube 4a, the video processor 6 and the universal cable 4b, or the video processor 6 and the remote control 13.

In the present embodiment, the RFID device 70 is located such that the device 70 transmits and receives data to and from the controller 318 of the endoscope washer-disinfector 3 via the RFID antenna 21a of the endoscope washer-disinfector 3 (refer to Fig. 7), when the endoscope-mounted tray 103 is loaded to the washer-disinfector 3.

In addition, similarly to the first embodiment, the RFID device 23 is incorporated within the base end part of the insertion tube 4a. In the following, to distinguish both RFID devices from one the other, the RFID device 23 of the insertion tube 4a is represented as a "scope RFID device 23," while the RFID 70 of the tray 103 is represented as a "tray RFID device 70." The remaining components are the same or similar as or to those in the first embodiment.

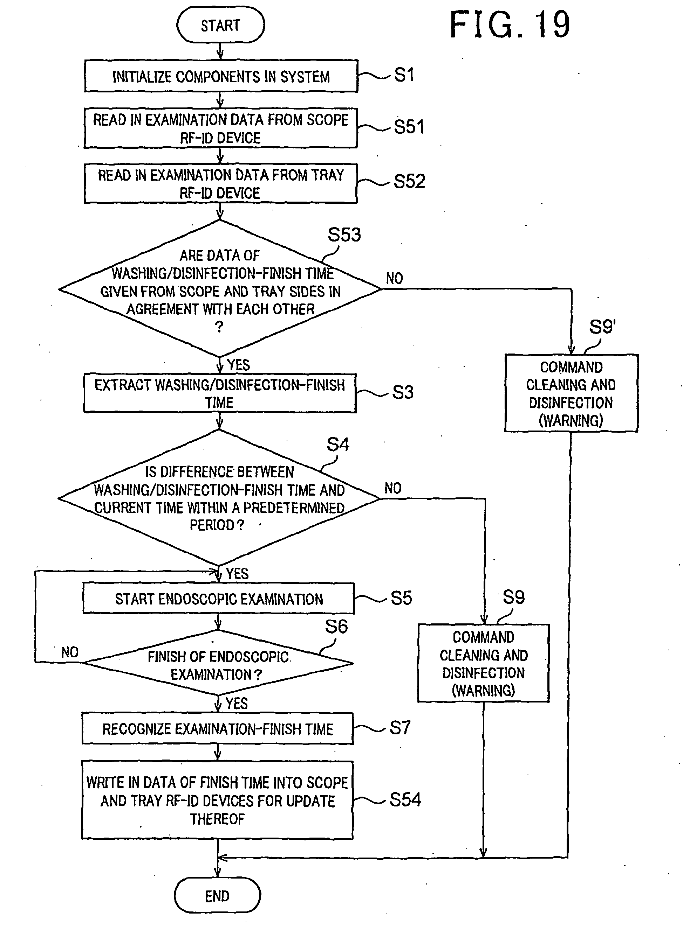

Referring to Figs. 19 - 22, the operations of the present embodiment will now be described. In the present embodiment, as described already, the insertion tube 4a is accommodated in the tray 103, and delivered and stored, with the insertion tube 4a still accommodated therein. In addition, with the insertion tube 4a accommodated in the tray 103, the tray 103 is loaded to the endoscope washer-disinfector 3. In such a loaded state, the insertion tube 4a is subjected to various repossessing steps including the brushing, cleaning and disinfecting steps.

In the endoscope system according to the present embodiment, when an endoscopic examination is needed, the tray 103 in which the insertion tube 4a is accommodated is placed on the work plate 9 (refer to Fig. 1), and then the insertion tube 4a is picked up from the tray 103. The empty tray 103 is left on the work plate 9.

And similarly to the first embodiment, prior to an endoscopic examination, an operator connects the universal cable 4b and the duct tube 4c to the insertion tube 4a so as to complete the endoscope 4. Further, the operator connects the universal cable 4b to the video processor 6 and the duct tube 4c to the air-supply water-supply unit 7. The operator also connects the remote control 13 to the video processor 6.

In this connected state, the video processor 6, light source 5, and air-supply water-supply unit 7 are put into operation by turning on the power, whereby the endoscope apparatus 2 is ready for an endoscopic examination.

The controller 609 of the video processor 6 (refer to Fig. 6) performs the initialization with the video processor 6 (step S1 in Fig. 19), before executing the processes at steps S51 to S53 which are inherent to the second embodiment.

Practically, at step S51, the controller 609 reads in examination data from the scope RFID device 23 via the RFID antenna 21 of the universal cable 4b (refer to Fig. 3). As a result, cleaning/disinfection-finish time data, which is obtained on the scope side, is extracted from the scope RFID device 23. Then at step S52, the controller 609 reads in examination data from the tray RFID device 70 via the RFID antenna 701 disposed on or near the work plate 9 and the arm 12, whereby cleaning/disinfection-finish time data, which is obtained on the tray side, is extracted.

At step S53, the controller 609 makes a comparison between the scope-side and tray-side cleaning/disinfection-finish time data extracted at steps S51 and S52. Practically it is determined whether or not a difference between the scope-side and tray-side time instants is within a predetermined period of time (e.g., 1 second). That is, the determination is made as to whether or not the scope-side and tray-side time instants can be regarded as being in agreement with each other.

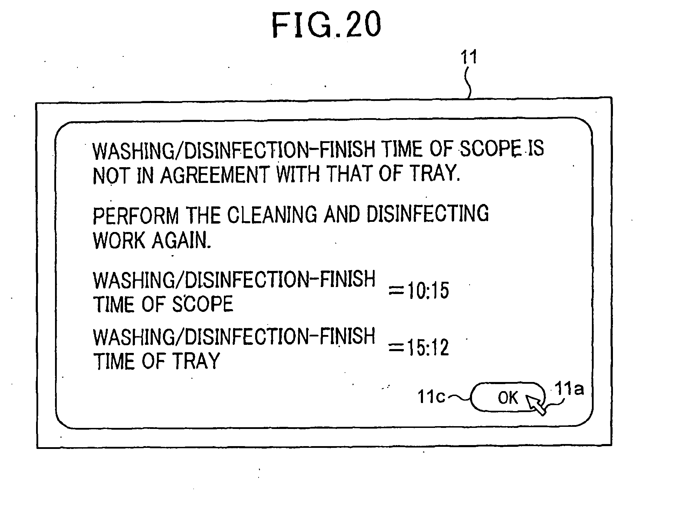

In cases where it is determined that both cleaning/disinfection-finish time instants are regarded as being in disagreement with each other because the difference between the scope-side and tray-side cleaning/disinfection-finish time instants reaches or exceeds the short predetermined period of time (NO at step S53), the controller 609 executes the process at step S9'. At this step, the controller 609 enables the monitor 11 to represent an error notice screen as pictorially shown in Fig. 20. This screen requests that an operator clean and disinfect the insertion tube 4a again, because bits of information about the finish time of the cleaning and disinfection steps mutually disagree. Clicking the "OK" button 11c on this error notice screen using the pointer 11a will terminate the processing.

In contrast, in cases it is determined at step S53 that the above difference is still within the short predetermined period of time (YES at step S53), i.e., it is regarded that both bits of temporal information agree with each other, the controller 609 moves its processing to step S3. The process at step S3 will be followed by steps S4 to S7, which are the same as those in the first embodiment.

Hence, the controller 609 recognizes current time data shown the current time, which is counted by the timer 607, as examination-finish time data (step S7), and then moves its processing to step S54 which is also inherent to the second embodiment. At this step S54, the controller 609 updates the examination-finish time by overwriting the recognized examination-finish time data in the scope RFID device 23 as the examination-finish time among the examination data by way of the RFID antenna 21 of the universal cable 4b. In addition, at this step S54, by way of the antenna 701 disposed on or near the work plate 9 or the arm 12, the controller 609 also writes the recognized examination-finish time in the tray RFID device 70. Then the processing is terminated.

In this way, prior to an endoscopic examination, the video processor 6 operates such that both cleaning/disinfection-finish time data stored in the insertion tube 4a and the tray 103 with the insertion tube 4a accommodated therein are compared with each other. And in cases both bits of temporal information differ from each other more than a predetermined threshold, a warning is issued. It is therefore avoidable that an endoscopic examination is erroneously performed using an insertion tube 4a having a cleaning/disinfection-finish time instant which is different from that of the tray 103.

There is a concern if the tray 103 differs from the insertion tube 4a in the cleaning/disinfection-finish time data. The reason is that there is a possibility that the insertion tube 4a is erroneously switched from the right tray to another tray during delivery or storage.

In this case, as shown in Fig. 20, the error notice screen is displayed for the warning. With the concerned insertion tube 4a sill kept in the tray 103, the operator is reminded that the insertion tube 4a should be cleaned and disinfected, because there is a doubt about erroneous use resulting from the disagreement in the time information. Hence it is steadily avoidable to use such a doubtful insertion tube 4a about the misuse, giving an improved reliability to the endoscopic examination.

In addition, similarly to the first embodiment, before starting an endoscopic examination, the cleaning/disinfection-finish time instant provided by the insertion tube 4a is compared in real time with the current time instant. For the examinations, it is therefore possible to use only insertion tubes 4a each of which has a time elapsing from the finish of the last cleaning and disinfection steps is still within the predetermined period of time, increasing the reliability of the examinations.

The cleaning and disinfecting steps for the insertion tube 4a used for endoscopic examinations will now be described.

As described in the first embodiment, an operator sets, into the washing bath 3a, the tray 103 with the insertion tube 4a accommodated within the tray 103, and turns on the power of the endoscope washer-disinfector 3. Responsively to this, the washer-disinfector 3 will start a series of programmed work steps including the cleaning and disinfecting steps.

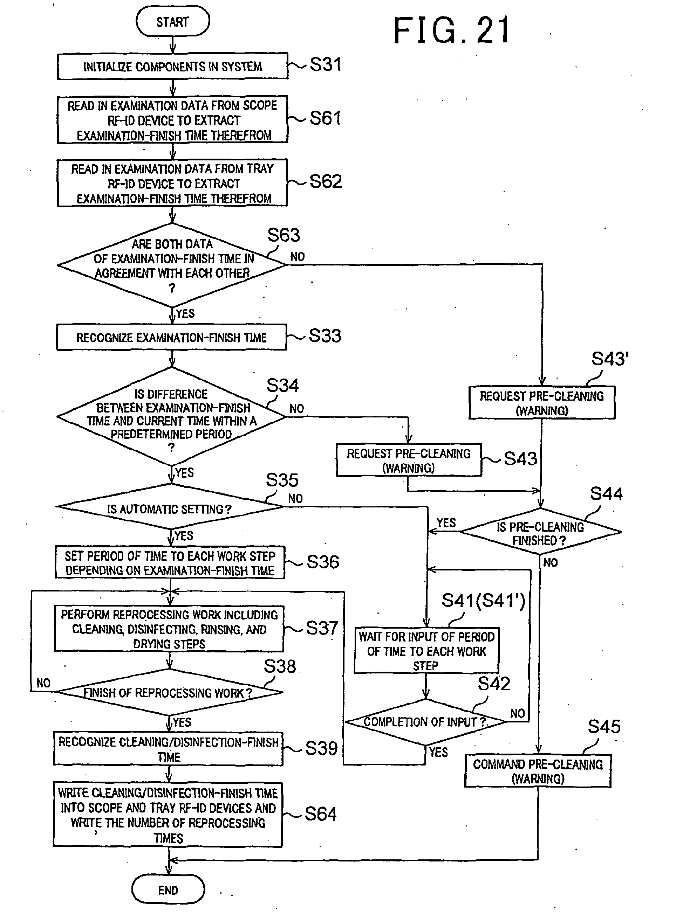

When the work steps are started, the controller 318 of the endoscope washer-disinfector 3 (refer to Fig. 8) initializes the washer-disinfector 3 at step S31 in Fig. 21.

The controller 318 proceeds to step S61, where, by way of the RFID antenna 21a of the endoscope washer-disinfector 3 (refer to Fig. 7), the controller 318 extracts examination-finish time data given on the scope side by reading examination data. Then at step S62, the controller 318 reads examination data from the tray RFID device 70 via the RFID antenna 21a of the washer-disinfector 3 to extract, from the examination data, examination-finish time data given on the tray side.

The controller 318 then proceeds to step S63, where the controller 318 make a comparison between the examination-finish time data given from the scope side at step S61 and the examination-finish time data given from the tray side at step S62. Practically a determination is made whether or not a difference between the examination-finish time instants given by both data is within a predetermined period of time (for example, 1 second), which is rather shorter. Thus this comparison shows whether or not both examination-finish time instants are (almost) in agreement with each other.

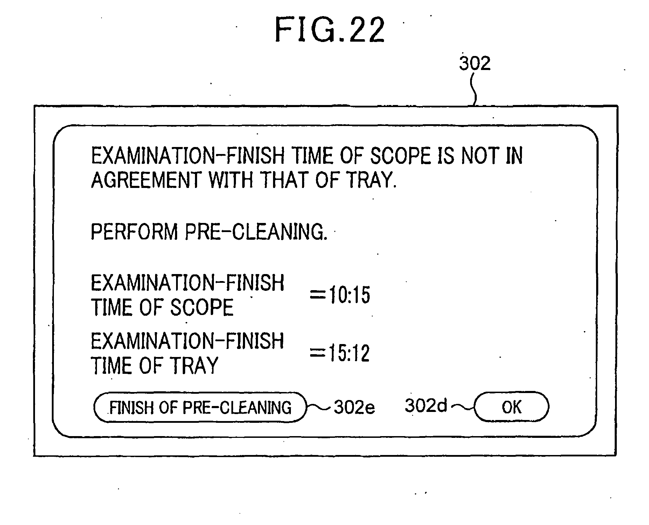

If it is determined at step S63 that both examination-finish time instants are in disagreement with each other due to the fact that the difference reaches or exceeds the predetermined period of time (YES at step S63), the controller 318 moves its processing to step S43'. At this step S43', as shown in Fig. 22, a pre-cleaning request screen is displayed on the LCD display unit 302 so that the screen massage points out an disagreement between the finish time instants of the endoscopic examination and request pre-cleaning serving as pre-reprocessing. Then the processing is moved to step S44.

Meanwhile if the determination is NO at step S63, i.e., the temporal difference is small and within the predetermined period of time, it is recognized that both examination-finish time instants agree with each other. In this case, the controller 318 moves to step S33, which is followed by steps S34 - S45. Steps S33 - S45 are the same as those in the first embodiment (refer to Fig. 11).

At step S39, the controller 318 recognizes current time data counted by the timer 316 as cleaning/disinfection-finish time data showing a time instant at which the work steps have finished, before proceeding to step S64. At step S64, via the RFID antenna 21a of the endoscope washer-disinfector 3 (refer to Fig. 7), the recognized cleaning/disinfection-finish time is written into the scope RFID device 23 and the tray RFID device 70, respectively. Concurrently with this, data showing the number of times of the cleaning and disinfecting steps is incremented. That is, those data are updated, and then the processing is terminated.

In the present embodiment, in this way, the examination-finish time data, i.e., finish time instants acquired on both the insertion tube and tray sides, are compared with each other. This compassion is made using the difference between both time instants. When it is found that the difference is larger than the threshold, i.e., the predetermined period of time, a warning is issued to notify the operator of the possibility that that there is a mismatch between the insertion tube 4a and the tray 103. In other words, the warning is made such that there is a concern about erroneously switchover of the insertion tube 4a from the right tray to another tray due to mismanagement of the storage or others. Hence, in such a case, it is requested that the insertion tube 4a is subject to a pre-cleaning step which is done by hand.

By performing the pre-cleaning step, the above concern about the erroneous switchovers can be eliminated.

The system according to the present embodiment is able to give a solution to the above concern by displaying the pre-cleaning request screen shown in Fig. 22 for a warning. By this screen, it is requested that an operator perform the pre-cleaning by hand, which is followed by the automatic cleaning and disinfecting steps based on operator's manually set periods of time. That is, in cases there is a concern about erroneous switchovers between trays and insertion tubes, the series of reprocessing steps including the cleaning and disinfecting steps are to be performed from the manual pre-cleaning, i.e., from the beginning, to be on the safe side.

Additionally, in the endoscope washer-disinfector 3, the examination-finish time instant of the insertion tube 4a, which is an objective being reprocessed, undergoes a comparison with the current time instant at which the reprocessing steps are to be performed, similarly to the first embodiment. As a result, if the comparison reveals that only a shorter period of time which can be ignored has passed from the finish of an endoscopic examination, the automatic reprocessing steps including the brushing, cleaning, and disinfecting steps are performed based on automatically or manually set periods of time. In contrast, if the comparison reveals that a longer period of time which cannot be ingoted has passed from the endoscopic examination finish, it is requested that the insertion tube 4a be again subjected to the reprocessing steps beginning from the manual re-cleansing step.

### (Third embodiment)

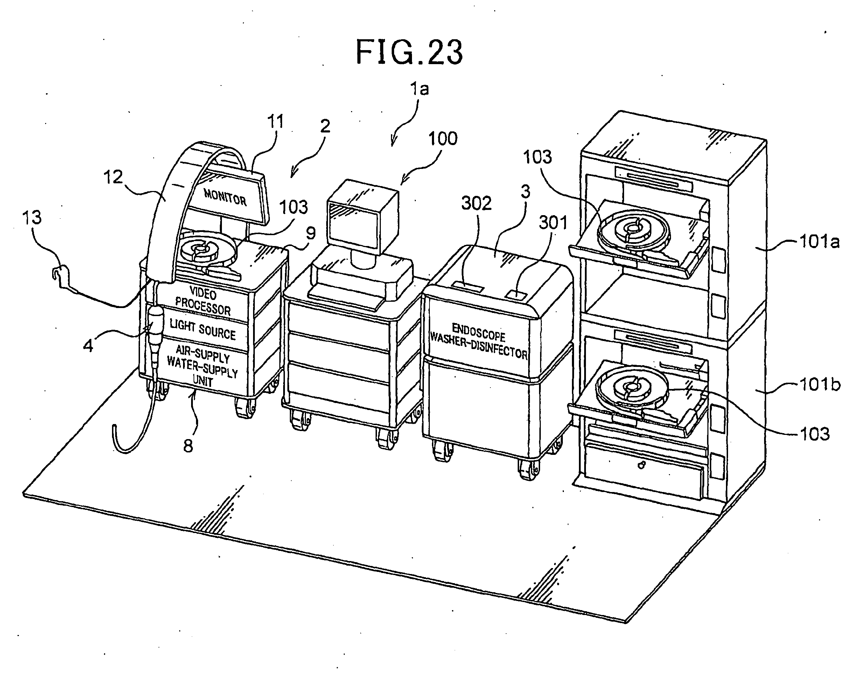

Referring to Figs. 23 to 30, a third embodiment according to the present embodiment will now be described. In the present embodiment, the identical or similar components to those in the first embodiment will be given the same reference numerals.

Fig. 23 shows an endoscope system 1a according to the present embodiment. This endoscope system 1a functionally includes a system for managing the cleaning and disinfecting work for endoscopes. As shown in Fig. 23, the endoscope system 1a is provided with an endoscope apparatus 2, an endoscope washer-disinfector 3, an endoscopic examination support apparatus 100, a first endoscope cabinet 101a, and a second endoscope cabinet 101b.

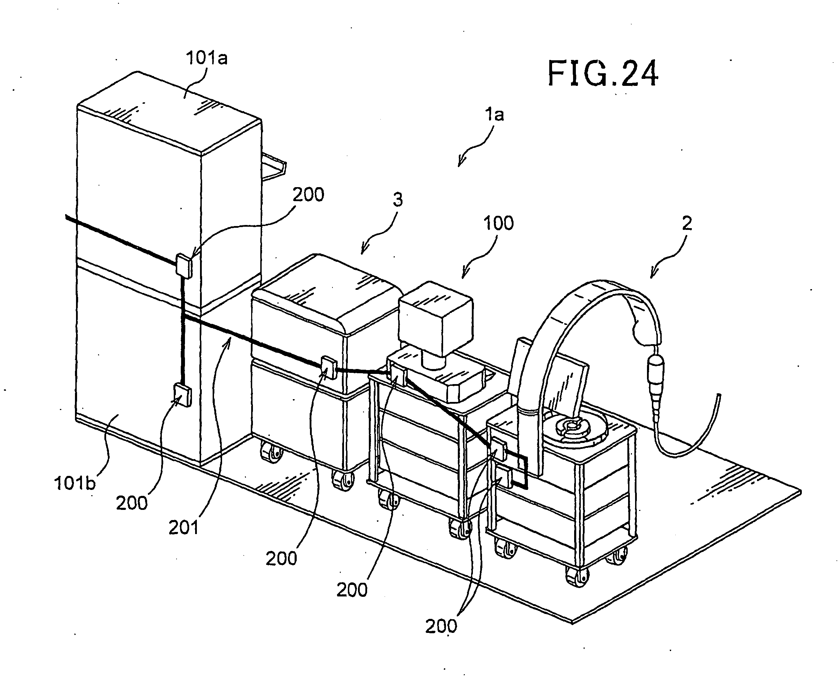

As shown in Fig. 24, a LAN (local area network) connector 200 is provided on each of the backs of the video processor 6, light source 5, endoscope washer-disinfector 3, endoscopic examination support apparatus 100, first cabinet 101a, and second cabinet 101b. These LAN connectors 200 are connected to each other through a LAN connection cable 201, whereby these equipments are linked with a in-house LAN.

The endoscopic examination support apparatus 100 is able to transmit and receive data to and from the video processor 6, endoscope washer-disinfector 3, first cabinet 101a, and second cabinet 101b via the LAN connectors 200 and LAN connection cable 201. Under such a communication environment, for supporting endoscopic examinations, this apparatus 100 has the capability of managing examination information about the work of endoscopic examinations (such as making and managing medical records, booking endoscopic examinations, recording examined histories, recording cleaning and disinfection histories, and managing history data) every scope ID. The scope ID is an ID of each endoscope and includes information about the type name, identification information such as manufacture's serial numbers, and information about duct characteristics.

Each of the first and second cabinets 101a and 101b is produced to store trays 103 therein, tray by tray, in which the insertion tube 4a is mounted in each tray 103. The tray 103 is the same as that described in the second embodiment.

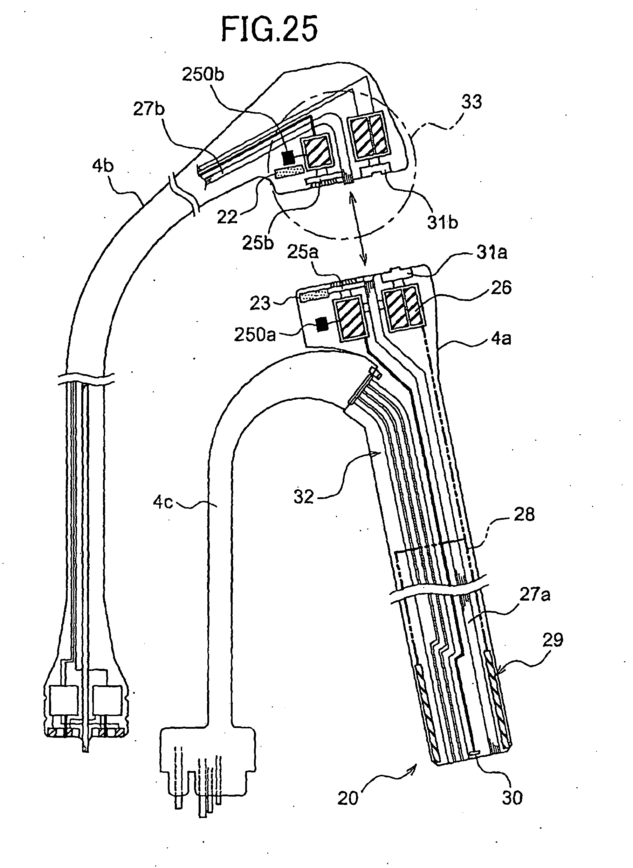

In the endoscope 4 communicably connected to the video processor 6, an ID memory 250a is disposed near the scope RFID 23 in the base end part of the insertion tube 4a, as shown in Fig. 25. The ID memory 250a is configured such that examination data can be written into the ID memory 250a and also read therefrom. This ID memory 250a is communicably connected to the video processor 6 via the connector 25a.

Similarly to the above, in the endoscope 4, an ID memory 250b is disposed in the distal end part of the universal cable 4b so that the ID memory 250b is located near the scope RFID device 22. The ID memory 250b is configured such that examination data can be written into the ID memory 250b and also read therefrom. This ID memory 250b is communicably connected to the video processor 6 via the connector 25b.

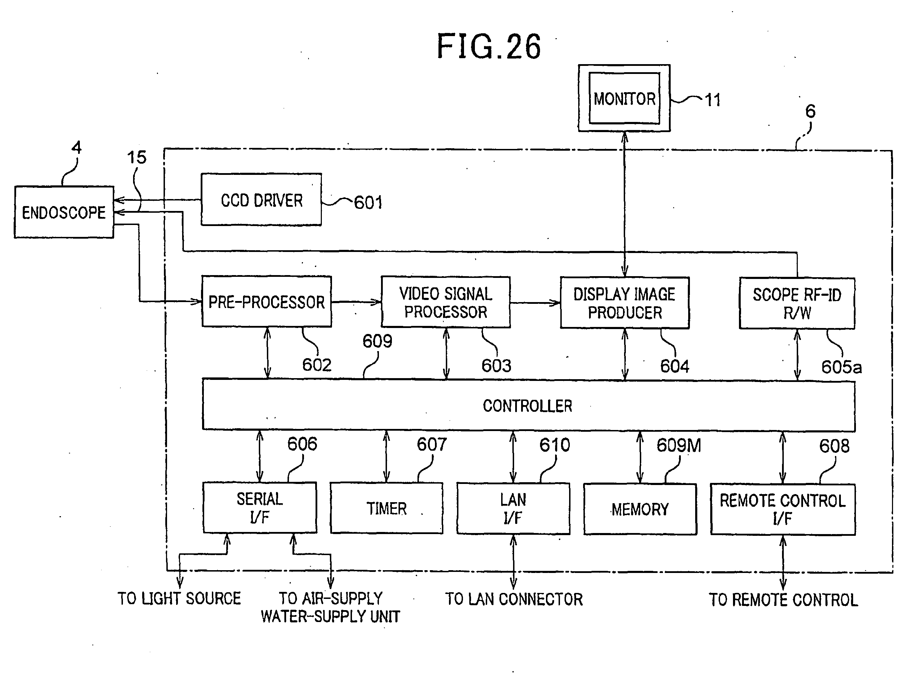

The video processor 6 according to the present embodiment is not provided the RFID R/W, differently from the foregoing one (refer to Fig. 6). In place of such an RFID R/W, the video processor 6 according to the present embodiment is provided with, as shown in Fig. 26, a scope ID R/W 605a electrically connected to the ID memories 250a and 250b for controlling examination data written and read from the ID memories 250a and 250b.

Further, the video processor 6 is provided with a LAN I/F 610 electrically connected to the LAN connector 200 for being network-connected to the in-house LAN.

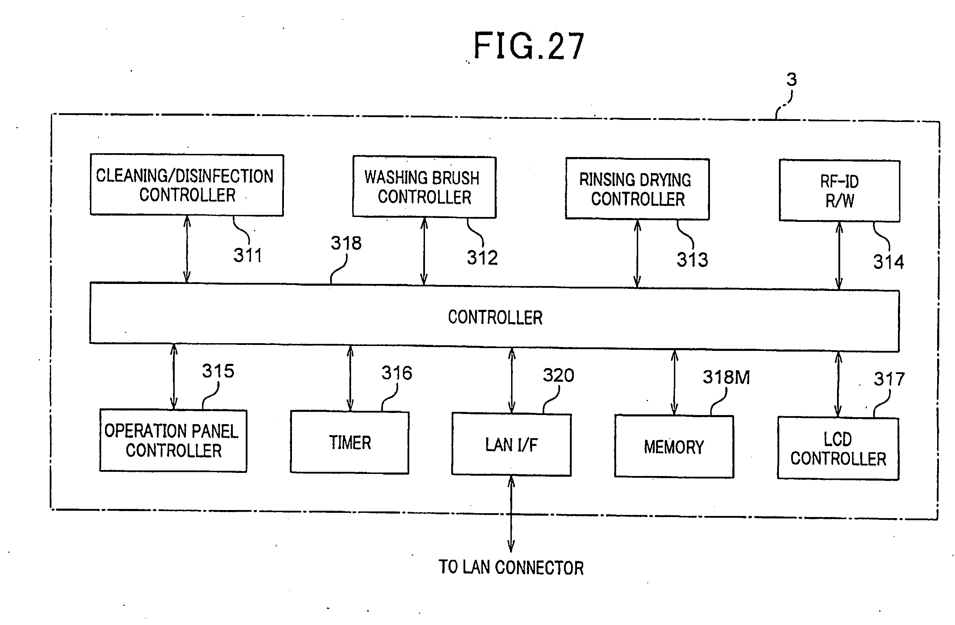

In addition, as shown in Fig. 27, the endoscope washer-disinfector 3 is provided with a LAN I/F 320 electrically connected to the LAN connector 200 for being network-connected to the in-house LAN.

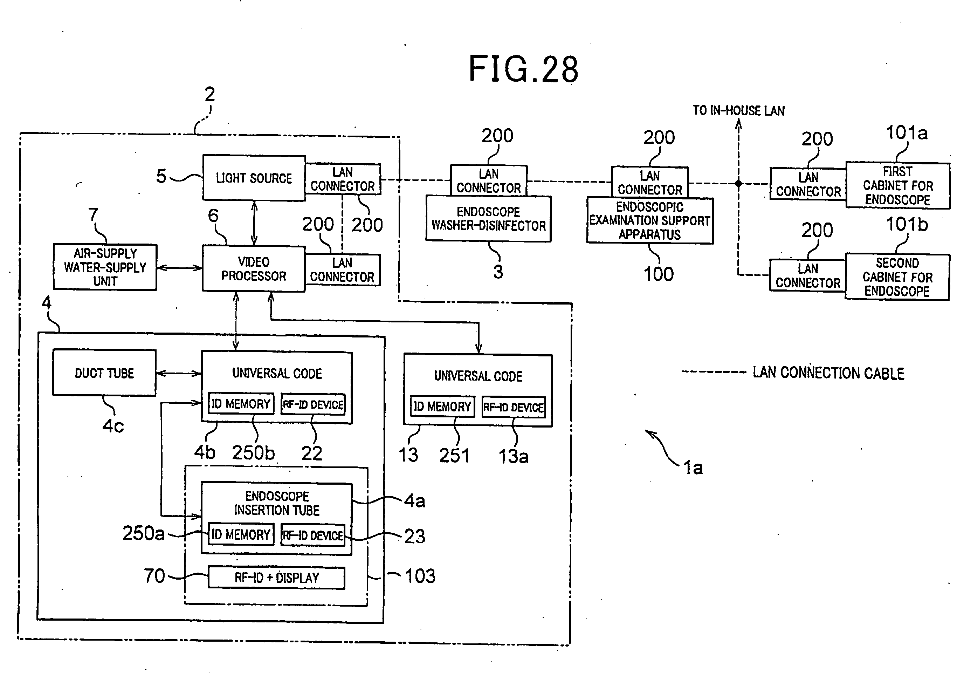

Fig. 28 shows connections in the endoscope system according to the present embodiment. As shown in Fig. 28, an ID memory 251 into and from which examination data is written and read is disposed in the remote control 13 connected to the video processor 6. The ID memory 251 is located near the RFID device 13a and electrically connected with the video processor 6. The remaining configurations are the same as those in the first embodiment.

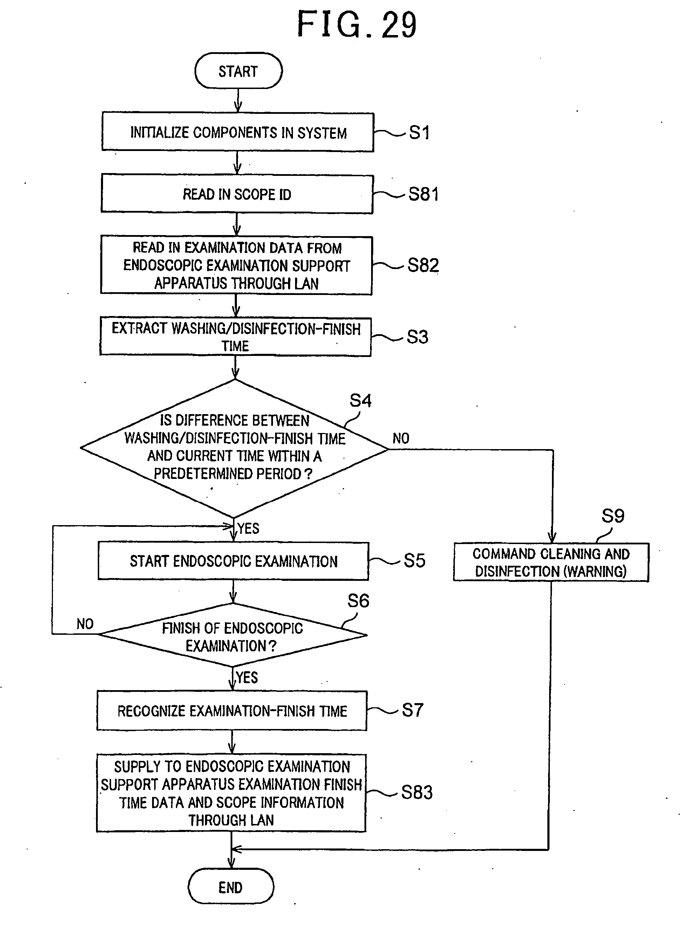

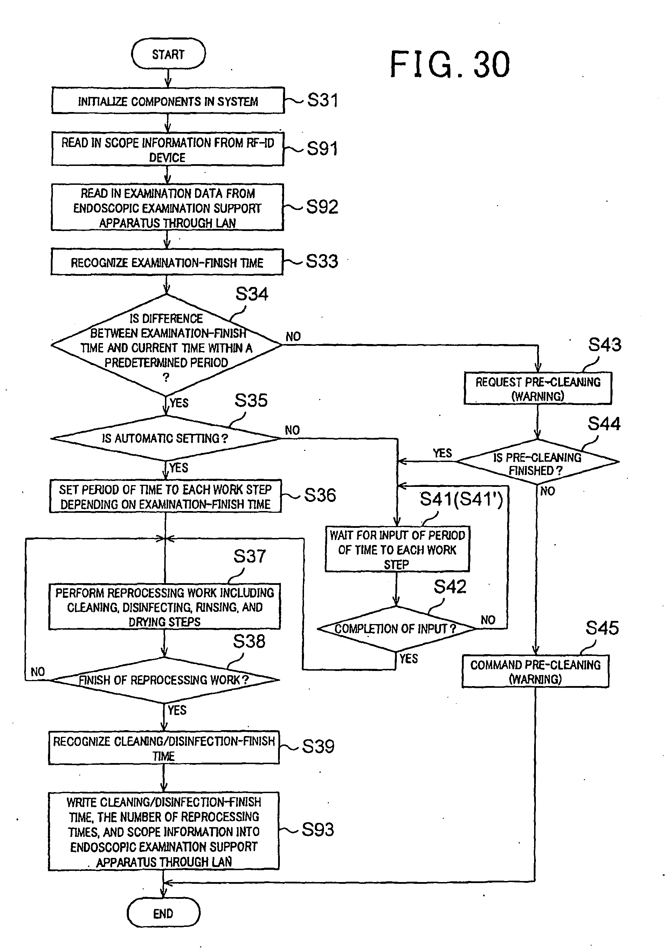

Referring to Figs. 29 and 30, the operations of the above endoscope system will now be described.

Prior to an endoscopic examination, the endoscope 4 is set up by an operator in the same way descried in the first and second embodiments, in which the connections to the air-supply water-supply unit 7 and the video processor 6 are made as described already. The power is also made on, so that it becomes possible to start the endoscopic examination using this endoscope apparatus 2.

Then the controller 609 of the video processor 6 starts performing a series of processes shown in Fig. 29, in which at step S1, the initialization is first made for the components of the video processor 6.

After this, the controller 609 proceeds to step S81, where the controller reads out examination data from the ID memory 250a via the scope ID R/W 605a to extract a scope ID from the examination data.

The controller 609 further proceeds to step S82 to be connected to the endoscopic examination support apparatus 100 via the LAN I/F 610. In this state, the controller 609 reads, from the endoscopic examination support apparatus 100, cleaning/disinfection-finish time data of an insertion tube corresponding to the read-out scope ID, before going to step S3,

The processes starting from step S3 are the same as those in the first embodiment except that step S8 is replaced by step S83.

After recognizing current time data showing a current time instant as examination-finish time data at step S7, the controller 609 performs the processing at step S83. In this processing, the controller 609 is connected to the endoscopic examination support apparatus 100 via the LAN I/F 610 to overwrite an examination-finish time instant among the examination data with the recognized examination-finish time data for updating the examination-finish time instant. Then the processing is terminated.

Referring to Fig. 30, the reprocessing work including the cleaning and disinfecting steps for the insertion tube 4a of the endoscope 4, which was used for the endoscopic examination, will now be described. This work is carried out by the endoscope washer-disinfector 3. Of course, the remaining parts of the endoscope 4 other than insertion tube 4a may also be subjected to the reprocessing work.

In the same way as the second embodiment, the tray 103 in which the used insertion tube 4a is accommodated is loaded to the washing bath 3a. Then the endoscope washer-disinfector 3 is powered on to start the reprocessing work including the cleaning and disinfecting steps.

As shown in Fig. 30, after the initialization at step S31, the controller 318 of the endoscope washer-disinfector 3 performs the processing at steps S91 and S92 in turns.

At step S91, the controller 318 reads out, via the RFID antenna 21a of the endoscope washer-disinfector 3 (refer to Fig. 7), a scope ID from the scope RFID device 23.

Then at step S92, the controller 318 connects to the endoscopic examination support apparatus 100 via the LAN I/F 320 to read out, from the apparatus 100, examination-finish time data of an insertion tube 4a corresponding to the read-out scope ID. And the controller 318 recognizes the read-out examination-finish time data as an examination-finish time instant, before going to step S33.

Step S33 is followed by steps S34 to S39 which are the same as those in the first embodiment, and step S39 is followed by step S93 which is inherent in the present embodiment.

At step S93, the controller 318 recognizes current time data showing a current time instant, which is counted by the timer 316, as cleaning/disinfection-finish time data. Then step S93, the controller 318 connects to the endoscopic examination support apparatus 100 via the LAN I/F 320 and overwrites the cleaning/disinfection-finish time among the examination data with the recognized cleaning/disinfection-finish time data in the support apparatus 100. This results in updating the cleaning/disinfection-finish time. Concurrently, at step S93, the data showing the number of times of reprocessing is also incremented for update. Then the processing is terminated.

In this way, the system of the present embodiment is able to provide the advantages identical to those gained in the first embodiment, and in addition, allows the endoscopic examination support apparatus 100 to manage the various types of work for endoscopic examinations every scope ID. Thus when endoscopes with existing ID memories and RFID devices are used, a conventional video processor with no RFID R/W (, which is for example the video processor 6 in the present embodiment) is still be available. Accordingly, without additionally installing a new RFID R/W, the time instant at which an endoscopic examination is finished can be obtained easily and less costly for a reliable cleanng and disinfecting work.

Incidentally some other forms of the foregoing embodiments may be provided. In the foregoing, the managing system according to the present invention is functionally realized by using, as hardware, the endoscope apparatus 2 and endoscope washer-disinfector 3, or additionally to those apparatuses 2 and 3, the endoscopic examination support apparatus 100. However this is merely an example. The managing system according to the present invention, which is in charge of performing the processing necessary for management of the cleaning and disinfecting steps, may be realized by another computer system other than exemplified. For example, a computer system dedicated to the processing shown in Figs. 9 and 11 may be arranged separately from the system shown in Figs. 1 and 23.

Although the description above contains many specificities, these should not be construed as limiting the scope of the invention but as merely providing illustrations of some of the presently preferred embodiments of the present invention. Thus the scope of the present invention should be determined by the appended claims.

## Claims

1. A managing system for managing not only an endoscope washer-disinfector (3) performing at least cleaning and disinfection with an insertion tube (4a) of an endoscope (4), the insertion tube (4a) being inserted into at least a duct of an object being examined, but also an endoscope apparatus (2) performing an endoscopic examination using the endoscope (4), the system comprising:
a first acquiring unit configured to acquire information indicative of a finish time of the endoscopic examination which is the newest and performed by the endoscope apparatus (2); and
a first manager configured to manage, at least, states of the cleaning and disinfection performed by the endoscope washer-disinfector (3) depending on the information indicative of the finish time of the endoscopic examination acquired by the first acquiring unit, **characterized in that**
the insertion tube (4a) is accommodated in a tray (103) for delivery, cleaning, disinfection, and storage, the system further comprising:
a second acquiring unit configured to acquire information indicative of a finish time of the cleaning and disinfection which is the newest and performed with the insertion tube (4a) by the endoscope washer-disinfector (3);
a second manager configured to manage, at least, the states of the cleaning and disinfection performed by the endoscope washer-disinfector (3) depending on the information indicative of the finish time of the cleaning and disinfection acquired by the second acquiring unit;
a third acquiring unit configured to acquire, as of the insertion tube (4a) and the tray (103), respectively, information indicative of the finish time of the endoscopic examination which is the newest and performed by the endoscope apparatus (2); and
a third manager configured to manage the states of at least the cleaning and disinfection performed by the endoscope washer-disinfector (3) depending on the information indicative of the finish time of the endoscope examination, the information being acquired by the third acquiring unit as of the insertion tube (4a) and the tray (103), respectively, wherein
the third manager comprises
first agreement determining means for determining whether or not two pieces of the finish time of the endoscopic examination as to the insertion tube (4a) and the tray (103), which are acquired by the third acquiring unit, are in agreement with each other, and
first command means for commanding re-performance of the cleaning and disinfection together with pre-cleaning, in cases where the first agreement determining means determines that there is no agreement between two pieces of the finish time of the endoscopic examination as to the insertion tube (4a) and the tray (103).

2. The managing system of claim 1, further comprising
a fourth acquiring unit configured to acquire, as to the insertion tube (4a) and the tray (103), respectively, information indicative of the finish time of the cleaning and disinfection performed for the insertion tube (4a) by the endoscope washer-disinfector (3); and
a fourth manager configured to perform management as to the cleaning and disinfection performed by the endoscope apparatus (2) depending on the information indicative of the finish time of the cleaning and disinfection as to the insertion tube (4a) and the tray (103), the information being acquired by the fourth acquiring unit.

3. The managing system of claim 2, wherein the fourth managing unit comprises
second agreement determining means for determining whether or not two pieces of the finish time of the cleaning and disinfection as to the insertion tube (4a) and the tray (103), which are acquired by the fourth acquiring unit, are in agreement with other, and
second command means for commanding re-performance of the cleaning and disinfection, in cases where the second agreement determining means determines that there is no agreement between two pieces of the finish time of the cleaning and disinfection as to the insertion tube (4a) and the tray (103).

## Patentansprüche

1. Verwaltungssystem zum Verwalten nicht nur einer Endoskop-Wasch-Desinfektionseinrichtung (3), die zumindest eine Reinigung und Desinfektion mit einem Einführrohr (4a) eines Endoskops (4) ausführt, wobei das Einführrohr (4a) in mindestens einen Gang eines gerade untersuchten Objekts eingeführt ist, sondern auch einer Endoskopvorrichtung (2), die unter Verwendung des Endoskops (4) eine endoskopische Untersuchung durchführt, wobei das System umfasst:
eine erste Ermittlungseinheit, die zum Ermitteln von Information ausgelegt ist, die eine Abschlusszeit der endoskopischen Untersuchung angibt, welche die letzte ist und von der Endoskopvorrichtung (2) durchgeführt wird; und
eine erste Verwaltungseinrichtung, die ausgelegt ist zum Verwalten von zumindest Stadien der von der Endoskop-Wasch-Desinfektionseinrichtung (3) durchgeführten Reinigung und Desinfektion in Abhängigkeit von der Information, welche die von der ersten Ermittlungseinheit ermittelte Abschlusszeit der endoskopischen Untersuchung angibt, **dadurch gekennzeichnet, dass**
das Einführrohr (4a) in einer Schale (103) zur Übergabe, Reinigung, Desinfektion und Lagerung untergebracht ist, wobei das System ferner umfasst:
eine zweite Ermittlungseinheit, die zum Ermitteln von Information ausgelegt ist, die eine Abschlusszeit der Reinigung und Desinfektion, welche die letzte ist und mit dem Einführrohr (4a) von der Endoskop-Wasch-Desinfektionseinrichtung (3) durchgeführt wird, angibt;
eine zweite Verwaltungseinrichtung, die ausgelegt ist zum Verwalten zumindest der Stadien der von der Endoskop-Wasch-Desinfektionseinrichtung (3) durchgeführten Reinigung und Desinfektion in Abhängigkeit von der Information, welche die von der zweiten Ermittlungseinheit ermittelte Abschlusszeit der Reinigung und Desinfektion angibt,
eine dritte Ermittlungseinheit , die zum Ermitteln von Information hinsichtlich des Einführrohrs (4a) und der Schale (103) ausgelegt ist, die eine Abschlusszeit der endoskopischen Untersuchung, welche die letzte ist und von der Endoskopvorrichtung (2) durchgeführt wird, angibt; und
eine dritte Verwaltungseinrichtung, die ausgelegt ist zum Verwalten der Stadien zumindest der von der Endoskop-Wasch-Desinfektionseinrichtung (3) durchgeführten Reinigung und Desinfektion in Abhängigkeit von der Information, welche die von der dritten Ermittlungseinheit ermittelte Abschlusszeit der endoskopischen Untersuchung angibt, wobei die Information von der dritten Ermittlungseinheit hinsichtlich des Einführrohrs (4a) bzw. der Schale (103) ermittelt wird, wobei
die dritte Verwaltungseinrichtung umfasst
erste Übereinstimmmungs-Bestimmungsmittel zum Bestimmen, ob zwei Stücke der Abschlusszeit der endoskopischen Untersuchung hinsichtlich des Einführrohrs (4a) und der Schale (103), die von der dritten Ermittlungseinheit ermittelt werden, miteinander übereinstimmen, und
erste Steuermittel zum Steuern einer nochmaligen Durchführung der Reinigung und Desinfektion mit einer Vorreinigung in Fällen, in denen das erste Übereinstimmungs-Bestimmungsmittel bestimmt, dass es keine Übereinstimmung zwischen zwei Stücken der Abschlusszeit der endoskopischen Untersuchung hinsichtlich des Einführrohrs (4a) und der Schale (103) gibt.

2. Verwaltungssystem nach Anspruch 1, ferner mit
einer vierten Ermittlungseinheit, die ausgelegt ist zum Ermitteln von Information hinsichtlich des Einführrohrs (4a) bzw. der Schale (103), welche die Abschlusszeit der von der Endoskop-Wasch-Desinfektionseinrichtung (3) für das Einführrohr (4a) durchgeführten Reinigung und Desinfektion angibt,
einer vierten Verwaltungseinrichtung, die eine Verwaltung ausführt hinsichtlich der von der Endoskopvorrichtung (2) durchgeführten Reinigung und Desinfektion in Abhängigkeit von der Information, welche die Abschlusszeit der Reinigung und Desinfektion hinsichtlich des Einführrohrs (4a) und der Schale (103) angibt, wobei die Information von der vierten Ermittlungseinheit ermittelt wird.

3. Verwaltungssystem nach Anspruch 2, wobei die vierte Verwaltungseinheit umfasst
zweite Übereinstimmungs-Bestimmungsmittel zum Bestimmen, ob zwei Stücke der Abschlusszeit der Reinigung und Desinfektion hinsichtlich des Einführrohrs(4) und der Schale (103), die von der vierten Ermittlungseinheit ermittelt werden, miteinander übereinstimmen oder nicht, und
zweite Steuermittel zum Steuern einer nochmaligen Durchführung der Reinigung und Desinfektion in Fällen, in denen das zweite Übereinstimmungs-Bestimmungsmittel bestimmt, dass es keine Übereinstimmung zwischen zwei Stücken der Abschlusszeit der Reinigung und Desinfektion hinsichtlich des Einführrohrs (4a) und der Schale (103) gibt.

## Revendications

1. Système de gestion pour gérer non seulement un dispositif de lavage-désinfection d'endoscope (3) effectuant au moins un nettoyage et une désinfection avec un tube d'insertion (4a) d'un endoscope (4), le tube d'insertion (4a) étant inséré dans au moins un conduit d'un objet étant examiné, mais également un appareil endoscopique (2) effectuant un examen endoscopique en utilisant l'endoscope (4), le système comprenant :
une première unité d'acquisition configurée pour acquérir une information indicatrice d'un moment de fin de l'examen endoscopique qui est le plus récent et effectué par l'appareil endoscopique (2) ; et
un premier gestionnaire configuré pour gérer, au moins, des états du nettoyage et de la désinfection effectués par le dispositif de lavage-désinfection d'endoscope (3) en fonction de l'information indicatrice du moment de fin de l'examen endoscopique acquise par la première unité d'acquisition, **caractérisé en ce que**
le tube d'insertion (4a) est reçu dans un plateau (103) pour délivrance, nettoyage, désinfection et stockage, le système comprenant en outre :
une deuxième unité d'acquisition configurée pour acquérir une information indicatrice d'un moment de fin du nettoyage et de la désinfection qui sont les plus récents et effectués avec le tube d'insertion (4a) par le dispositif de lavage-désinfection d'endoscope (3) ;
un deuxième gestionnaire configuré pour gérer, au moins, les états du nettoyage et de la désinfection effectués par le dispositif de lavage-désinfection d'endoscope (3) en fonction de l'information indicatrice du moment de fin du nettoyage et de la désinfection acquise par la deuxième unité d'acquisition ;
une troisième unité d'acquisition configurée pour acquérir, pour ce qui concerne le tube d'insertion (4a) et le plateau (103), respectivement, une information indicatrice du moment de fin de l'examen endoscopique qui est le plus récent et effectué par l'appareil endoscopique (2) ; et
un troisième gestionnaire configuré pour gérer les états d'au moins le nettoyage et la désinfection effectués par le dispositif de lavage-désinfection d'endoscope (3) en fonction de l'information indicatrice du moment de fin de l'examen endoscopique, l'information étant acquise par la troisième unité d'acquisition pour ce qui concerne le tube d'insertion (4a) et le plateau (103), respectivement, dans lequel
le troisième gestionnaire comprend
un premier moyen de détermination d'accord pour déterminer si deux éléments du moment de fin de l'examen endoscopique pour ce qui concerne le tube d'insertion (4a) et le plateau (103), qui sont acquis par la troisième unité d'acquisition, sont ou non en accord l'un avec l'autre, et
un premier moyen de commande pour commander une nouvelle exécution du nettoyage et de la désinfection en même temps qu'un pré-nettoyage, dans des cas où le premier moyen de détermination d'accord détermine qu'il n'y a pas d'accord entre deux éléments du moment de fin de l'examen endoscopique pour ce qui concerne le tube d'insertion (4a) et le plateau (103).

2. Système de gestion selon la revendication 1, comprenant en outre
une quatrième unité d'acquisition configurée pour acquérir, pour ce qui concerne le tube d'insertion (4a) et le plateau (103), respectivement, une information indicatrice du moment de fin du nettoyage et de la désinfection effectués pour le tube d'insertion (4a) par le dispositif de lavage-désinfection d'endoscope (3) ; et
un quatrième gestionnaire configuré pour effectuer une gestion pour ce qui concerne le nettoyage et la désinfection effectués par l'appareil endoscopique (2) en fonction de l'information indicatrice du moment de fin du nettoyage et de la désinfection pour ce qui concerne le tube d'insertion (4a) et le plateau (103), l'information étant acquise par la quatrième unité d'acquisition.

3. Système de gestion selon la revendication 2, dans lequel la quatrième unité de gestion comprend
un deuxième moyen de détermination d'accord pour déterminer si deux éléments du moment de fin du nettoyage et de la désinfection pour ce qui concerne le tube d'insertion (4a) et le plateau (103), qui sont acquis par la quatrième unité d'acquisition, sont ou non en accord l'un avec l'autre, et
un deuxième moyen de commande pour commander une nouvelle exécution du nettoyage et de la désinfection, dans des cas où le deuxième moyen de détermination d'accord détermine qu'il n'y a pas d'accord entre deux éléments du moment de fin du nettoyage et de la désinfection pour ce qui concerne le tube d'insertion (4a) et le plateau (103).
